# EUROPÄISCHE PATENTSCHRIFT

(11) **EP 2 091 958 B1**
(45) Veröffentlichungstag und Bekanntmachung des Hinweises auf die Patenterteilung: **27.04.2011**
(21) Anmeldenummer: 07822522.4
(22) Anmeldetag: 13.11.2007
(51) Int. Cl.: C07F 9/6574, C07B 41/06

(54) **BISPHOSPHITLIGANDEN FÜR DIE ÜBERGANGSMETALLKATALYSIERTE HYDROFORMYLIERUNG**
BISPHOSPHITE LIGANDS FOR HYDROFORMYLATION CATALYZED BY TRANSITION METALS
LIGANDS BIPHOSPHITE POUR L'HYDROFORMYLATION CATALYSÉE PAR DES MÉTAUX DE TRANSITION

(30) Priorität: 13.12.2006 DE 102006058682
(43) Veröffentlichungstag der Anmeldung: 26.08.2009
(73) Patentinhaber: Evonik Oxeno GmbH, 45772 Marl (DE)
(72) Erfinder: SELENT, Detlef, 18059 Rostock (DE); BÖRNER, Armin, 18059 Rostock (DE); KREIDLER, Burkard, 45657 Recklinghausen (DE); HESS, Dieter, 45770 Marl (DE); WIESE, Klaus-Diether, 45721 Haltern am See (DE)
(86) Internationale Anmeldenummer: PCT/EP2007/062248
(87) Internationale Veröffentlichungsnummer: WO 2008/071508

(56) Entgegenhaltungen:
- EP-A1- 0 472 071
- WO-A1-2007/114445
- JP-A- 11 292 887

## Beschreibung

Die vorliegende Erfindung betrifft Bisphosphite der allgemeinen Formel I, ein Verfahren zu ihrer Herstellung sowie die Verwendung dieser Bisphosphite in katalytischen Reaktionen.

Die Reaktion zwischen Alkenen (Olefinen), Kohlenmonoxid und Wasserstoff in Gegenwart eines Katalysators zu den um ein Kohlenstoffatom reicheren Aldehyden wird als Hydroformylierung (Oxierung) bezeichnet. Als Katalysatoren in diesen Reaktionen werden häufig Übergangsmetallverbindungen der VIII. Gruppe des Periodensystems der Elemente verwendet, insbesondere Verbindungen des Rhodiums und des Kobalts. Die Hydroformylierung mit Rhodium bietet im Vergleich zur Verwendung von Kobaltkatalysatoren in der Regel den Vorteil höherer Aktivität und ist somit zumeist wirtschaftlicher. Bei der durch Rhodium katalysierten Hydroformylierung werden zumeist Komplexe eingesetzt, die aus Rhodium und bevorzugt aus trivalenten Phosphorverbindungen als Liganden bestehen. Bekannte Liganden sind beispielsweise solche aus den Klassen der Phosphane (Phosphine), Phosphinite, Phosphonite und Phosphite. Eine gute Übersicht über den Stand der Hydroformylierung von Olefinen findet sich in CORNILS, B., HERRMANN, W. A., "Applied Homogeneous Catalysis with Organometallic Compounds", Vol. 1&2, VCH, Weinheim, New York 1996 und in WIESE, K.-D., OBST, D., in: Top. Organomet. Chem. Vol. 18, Springer Verlag 2006, herausgegeben von Matthias Beller, sowie speziell zur Rhodium-katalysierten Hydroformylierung: VAN LEEUWEN, P., CLAVER, C. "Rhodium catalyzed hydroformylation", Springer 2000 und VAN LEEUWEN, P. "Homogeneous Catalysys: Understanding the Art", Springer 2005.

Die Wahl des Katalysatorsystems (Kobalt oder Rhodium sowie geeignete Liganden) ist jeweils vom gewählten Substrat und der gewünschten Zielverbindung abhängig. Hierzu seien im Folgenden einige Beispiele gegeben:

Den am häufigsten anzutreffenden Liganden stellt Triphenylphosphan, gegebenenfalls in einer durch Substitution mit polaren Gruppen gebildeten wasserlöslichen Form, dar, welcher in einem hohen Ligand/Rhodium-Verhältnis eingesetzt werden muss, um α-Olefine bei vergleichsweise niedrigen Drücken mit hoher Selektivität zu endständigen, also zu n-Aldehyden zu hydroformylieren.

Bisphosphanliganden und ihr Einsatz in der Hydroformylierung von Olefinen bei niedrigen Synthesegasdrücken werden beispielsweise in US 4,694,109 und US 4,879,416 beschrieben. In WO 95/30680 wird die Verwendung von zweizähnigen Phosphanliganden in katalytischen Reaktionen, darunter auch Hydroformylierungsreaktionen, offen gelegt. In den Patentschriften US 4,169,861, US 4,201,714 und US 4,193,943 werden ferrocenverbrückte Bisphosphane als Liganden für die Hydroformylierung beschrieben.

Auch Monophosphite stellen geeignete Liganden für die rhodiumkatalysierte Hydroformylierung verzweigter Olefine mit innenständigen Doppelbindungen dar, allerdings bei geringer Selektivität für im Regelfall gewünschte endständig hydroformylierte Verbindungen.

EP 0 155 508 beschreibt die Verwendung bisarylensubstituierter Monophosphite bei der rhodiumkatalysierten Hydroformylierung sterisch gehinderter Olefine, z. B. von Isobuten.

Rhodium-Bisphosphit-Komplexe katalysieren die Hydroformylierung linearer Olefine mit end- und innenständigen Doppelbindungen, wobei überwiegend endständige Aldehyde entstehen, Selektivitäten von mehr als 95 % jedoch außergewöhnlich sind. Solche Phosphite sind im Regelfall sehr hydrolyselabil. Der Einsatz substituierter Bisaryldiole als Edukte für Phosphitliganden erbrachte erhebliche Verbesserungen, wie in EP 0.214 622 oder EP 0 472 071 beschrieben.

JP 11-292887 beschreibt die Herstellung von Bisphosphiten und deren Einsatz bei der Hydroformylierung.

Häufige Neben- oder Folgereaktionen in der metalllcatalysierten Hydroformylierungsreaktion sind die Hydrierung, etwa von Olefinen zu Alkanen oder von Aldehyden zu Alkoholen sowie die Isomerisierung von Doppelbindungen. In EP 1 201 675 und US 2006100453 macht man sich letzteres zu Nutze, um mittels eines O-Acylphosphitliganden interne Alkene bevorzugt zunächst zu endständigen Olefinen zu isomerisieren und diese dann endständig, also zu n-Aldehyden zu hydroformylieren. EP 0 472 071 beschreibt strukturell ähnliche Rhodium-Bisphosphitkatalysatoren, die in der Hydroformylierung von 2-Buten hohe Isomerisierungsaktivitäten bei moderaten n-Selektivitäten zeigen. Diese Isomerisierungsreaktion ist jedoch mitunter unerwünscht, da umgekehrt auch endständige Olefine zu internen Alkenen isomerisieren, wobei letztere die thermodynamisch stabileren Spezies darstellen. Eine nicht isomerisierende Ligandenklasse stellen von B. Breit et al. in Chem. Eur. J. 2006, 12, 6930-6939 vorgestellte Phosphabarrelene dar. Mit diesen wurde 2-Octen weitgehend isomerisierungsfrei zu 2-Methyloctanal und 2-Ethylheptanal mit einer Regioselek.rtivität von lediglich 62 : 38 umgesetzt. Die Hydroformylierung endständiger Olefine wurde dort nicht beschrieben.

Im Regelfall sind jedoch die endständig hydroformylierten Produkte erwünscht. So weisen beispielsweise Weichmacher (z. B. Phthalate), die aus Alkoholen, die durch endständige Hydroformylierung von Olefinen und anschlieliende Hydrierung gewonnen werden, bei der Verwendung in Kunststoffen (z. B. in PVC) günstigere Eigenschaften auf, etwa hinsichtlich der Elastizität, als Weichmacher auf Basis von verzweigten Aldehyden.

Auch die Hydroformylierung von Verbindungen mit mehreren Doppelbindungen erfordert zumeist nicht isomerisierende, hochselektive Katalysatoren, um zu einem diskreten (einzelnen) Produkt zu gelangen.

Ausgehend von den in EP 0.472 071 beschriebenen Rhodium-Bisphosphitkatalysatoren bestand deshalb die Aufgabe der vorliegenden Erfindung, alternative Bisphosphitliganden bereitzustellen, die vorzugsweise eine terminale Hydroformylierung von endständigen Doppelbindungen (also eine endständige Hydroformylierung, die zu Aldehyden mit einem hohen n/iso-Verhältnis führt) begünstigen und besonders bevorzugt eine geringe Isomerisierungsaktivität aufweisen. Die endständigen Doppelbindungen sollen vorzugsweise mit einem sehr hohen n/iso-Verhältnis hydroformyliert werden. Des Weiteren sollten die erfindungsgemäßen Bisphosphitliganden vorzugsweise eine höhere Hydrolysestabilität als die in EP 0 472 071 beschriebenen aufweisen.

Überraschenderweise wurde gefunden, dass diese Aufgabe durch Bisphosphitliganden der Formel I 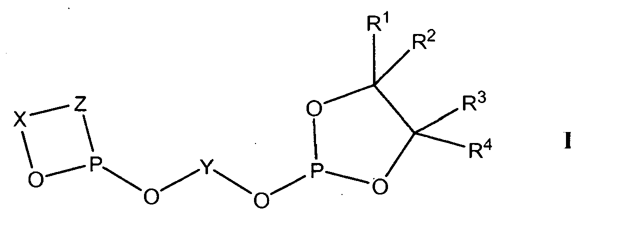 mit
X = einem zweiwertigen substituierten oder unsubstituierten Bisalkylen- oder Bisarylenrest, der ein oder mehrere Heteroatom(e) enthalten kann,
Y = einem zweiwertigen substituierten oder unsubstituierten Bisarylen- oder Bisalkylenrest, der ein oder mehrere Heteroatom(e) enthalten kann,
Z = Sauerstoff oder NR⁹,
R¹, R², R³, R⁴ gleiche oder verschiedene, substituierte oder unsubstituierte, verknüpfte, unverknüpfte, kondensierte oder unkondensierte Aryl- oder Heteroarylreste und R⁹ = Wasserstoff oder substituierter oder unsubstituierter Alkyl- oder Arylrest, der ein oder mehrere Heteroatom(e) enthalten kann, gelöst wird.

Gegenstand der vorliegenden Erfindung sind deshalb Bisphosphite der Formel I 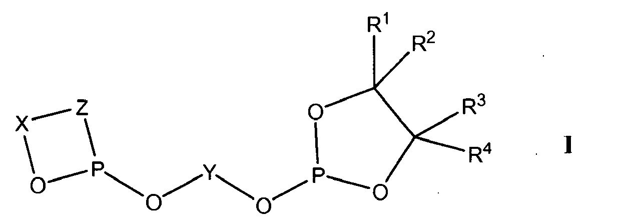 mit
X = einem zweiwertigen substituierten oder unsubstituierten Bisalkylen- oder Bisarylenrest, der auch Heteroatome enthalten kann,
Y = einem zweiwertigen substituierten oder unsubstituierten Bisarylen- oder Bisalkylenrest,
Z = Sauerstoff oder NR⁹,
R¹, R², R³, R⁴ gleiche oder verschiedene, substituierte oder unsubstituierte, verknüpfte, unverknüpfte, kondensierte oder unkondensierte Aryl- oder Heteroarylreste,
und R⁹ = Wasserstoff oder substituierter oder unsubstituierter Alkyl- oder Arylrest,
sowie Metallkomplexe, die ein solches Bisphosphit der Formel I als Liganden aufweisen.

Ebenfalls Gegenstand der vorliegenden Erfindung ist ein Verfahren zur Herstellung der Bisphosphite der Formel **I.**

Außerdem ist Gegenstand der vorliegenden Erfindung die Verwendung eines Bisphosphits der Formel I oder eines Metallkomplexes, der ein Bisphosphit der Formel **I** enthält, in katalytischen Reaktionen, insbesondere in der Hydroformylierung.

Die erfindungsgemäßen Verbindungen weisen folgende Vorteile auf Gegenüber den in EP 0 472 071 beschrieben Liganden weisen die erfindungsgemäßen Bisphosphite bei der Verwendung als Liganden in der Hydroformylierung eine geringere Isomerisierungsaktivität auf. Zudem werden mit Katalysatoren, die aus den erfindungsgemäßen Liganden gebildet werden, hochselektiv endständige Doppelbindungen hydroformyliert, wohingegen innenständige Doppelbindungen nahezu unangetastet bleiben. So können beispielsweise bei der Hydroformylierung von Olefinen, die sowohl innen- als auch endständige Doppelbindungen aufweisen, mit hoher Selektivität die endständigen Doppelbindungen hydroformyliert werden. Oder es können aus einem Olefingemisch heraus selektiv die Bestandteile, die endständige Doppelbindungen tragen, hydroformyliert werden. Die endständigen Doppelbindungen werden hochselektiv terminal, also mit einem hohen n/iso-Verhältnis hydroformyliert. Des Weiteren zeigen die erfindungsgemäßen Liganden eine hohe Stabilität, insbesondere gegenüber wässriger Hydrolyse.

Das erfindungsgemäße Bisphosphit weist die Formel I, 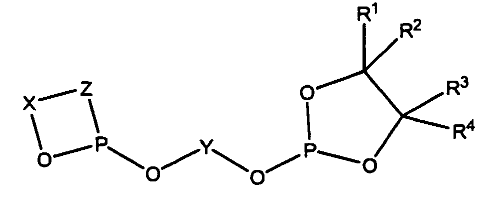 mit X = zweiwertiger substituierter oder unsubstituierter Bisalkylen- oder Bisarylenrest, der ein oder mehrere Heteroatom(e) enthalten kann, Y = zweiwertiger substituierter oder unsubstituierter Bisarylen- oder Bisalkylenrest, der ein oder mehrere Heteroatom(e) enthalten kann, Z = Sauerstoff oder NR⁹, R¹, R² , R³, R⁴ gleiche oder verschiedene, substituierte oder unsubstituierte, verknüpfte, unverknüpfte, kondensierte oder unkondensierte Aryl- oder Heteroarylreste, und R⁹ = Wasserstoff oder substituierter oder unsubstituierter Alkyl- oder Arylrest, der ein oder mehrere Heteroatom(e) enthalten kann, auf. Substituiert können die Reste R¹, R² , R³, R⁴, R⁹, X oder Y z. B. sein mit mindestens einem Rest, ausgewählt aus aliphatischen, alicyclischen, aromatischen, heteroaromatischen, gemischt aliphatisch-alicyclischen, gemischt aliphatischaromatischen, heterocyelischen, gemischt aliphatisch-heterocyclischen Kohlenwasserstoffresten mit 1 bis 50 Kohlenstoffatomen, F, Cl, Br, I, -CF₃, -(CH₂)ᵢ(CF₂)ⱼCF₃ mit i = 0 - 9 und j = 0 - 9, - SiR²¹ 3, -Si(OR²¹)₃ -SiR²¹(OR²¹)₂, -SiR²1₂OR²¹, -OSiR²¹ 3, -OSi(OR²¹)_{3'} -OSiR²¹(OR²¹)₂, - OSiR²¹₂OR²¹, -OR¹⁹, -COR¹⁹, -CO₂R¹⁹, -CO₂M, -SO₂R¹⁹, -SOR¹⁹, -SO₃R¹⁹, -SO₃M, -SO₂NR¹⁹R²⁰, -NR¹⁹R²⁰, oder -N=CR¹⁹R²⁰, wobei R¹⁹, R²⁰ und R²¹ unabhängig voneinander aus H, einwertigen substituierten oder unsubstituierten aliphatischen und aromatischen Kohlenwasserstoffresten mit 1 bis 25 Kohlenstoffatomen ausgewählt sind, wobei jedoch R²¹ = H ausgeschlossen ist und M ein Alkalimetall-, formal ein halbes Erdalkalimetall-, Ammonium- oder Phosphoniumion ist. Bevorzugte Substituenten, insbesondere für die Reste X und Y sind tert.-Butyl- und Methoxy-Gruppen. Die Reste R¹, R², R³, R⁴ sind vorzugsweise unsubstituierte Phenylreste. Solche Reste können z. B. solche sein, wie sie in den Formeln **I-1, I-2** oder **I-3** vorhanden sind. 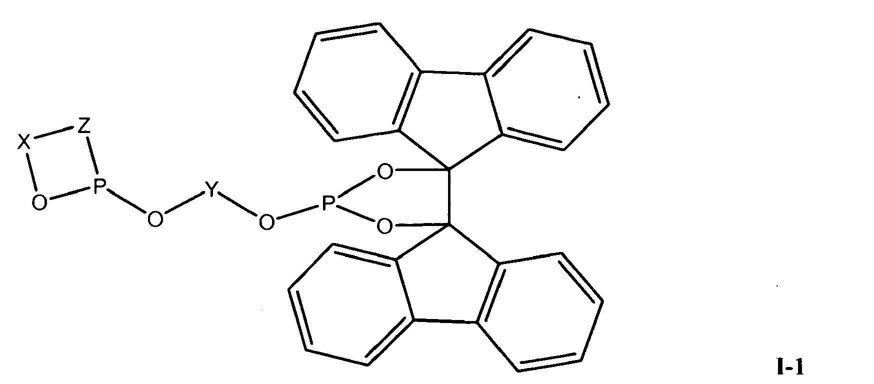 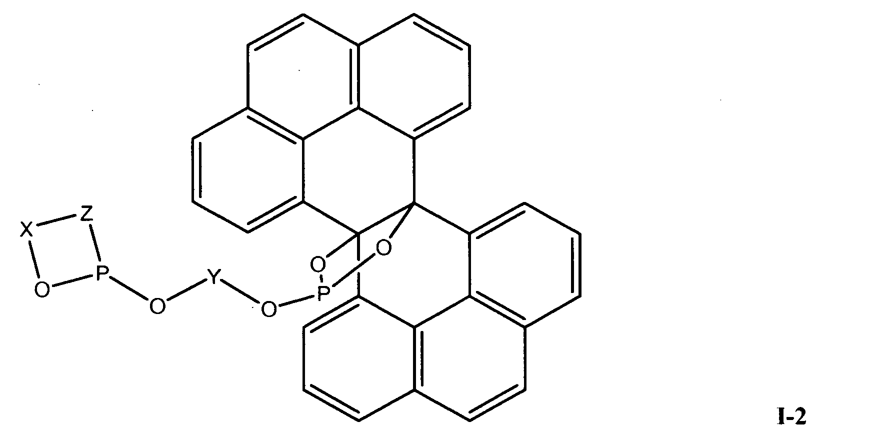 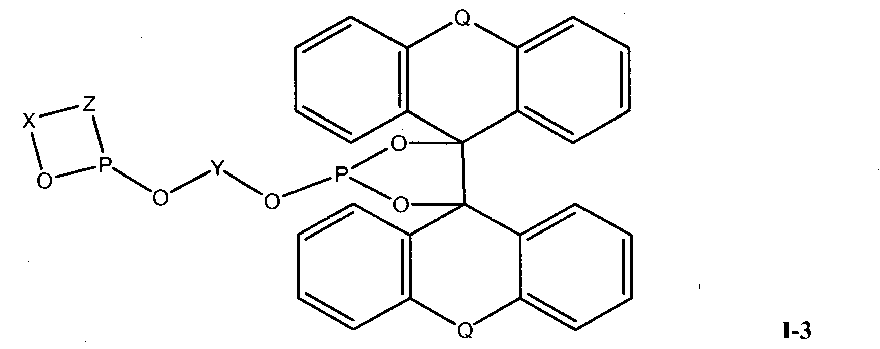 wobei Q z. B. gleich oder verschieden CH₂, CR⁹R¹⁰, CHR⁹, O, NH oder NR⁹ sein kann, wobei R⁹ und R¹⁰ gleich oder unterschiedlich sein können und die oben für R⁹ angegebene Bedeutung haben kann.

In dem erfindungsgemäßen Bisphosphit kann der Rest X insbesondere ein Rest **Xa** sein,

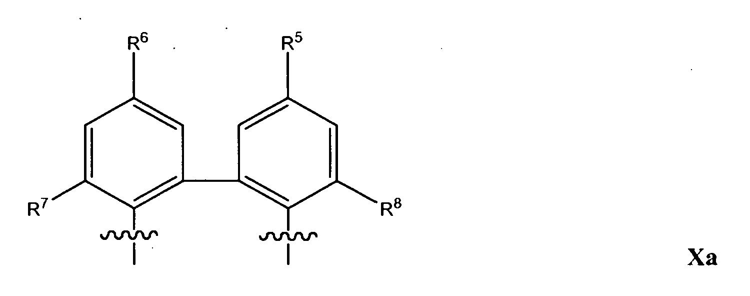

Die Reste R⁵, R⁶, R⁷, R⁸ können unabhängig voneinander substituierte oder unsubstituierte aliphatische, alicyclische, aromatische, heteroaromatische, gemischt aliphatisch-alicyclische, gemischt aliphatisch-aromatische, heterocyclische, gemischt aliphatisch-heterocyclische Kohlenwasserstoffreste mit 1 bis 50 Kohlenstoffatomen oder H, F, Cl, Br, I, -CF₃, -(CH₂)ᵢ(CF₂)ⱼCF₃ mit i = 0 - 9 und j = 0 - 9, -SiR²¹ 3, -Si(OR²¹)₃, -SiR²¹(OR²¹)₂, -SiR²¹2OR²¹, - OSiR²¹₃, -OSi(OR²¹)₃, -OSiR²¹(OR²¹)₂, -OSiR²¹₂OR²¹, -OR¹⁹, -COR¹⁹, -CO₂R¹⁹ -CO₂M, -SO₂R¹⁹, SOR¹⁹ , - SO₃ R¹⁹ , - SO₃M, - SO₂ NR¹⁹R²⁰ , - NR¹⁹R²⁰ oder N = CR¹⁹R²⁰, sein, wobei R¹⁹ , R²⁰ und R²¹ unabhängig voneinander aus H, einwertigen substituierten oder unsubstituierten aliphatischen und aromatischen Kohlenwasserstoffresten mit 1 bis 25 Kohlenstoffatomen ausgewählt sind, wobei jedoch R²¹ = H ausgeschlossen ist und M ein Alkalimetall-, formal ein halbes Erdalkalimetall-, Ammonium- oder Phosphoniumion ist. Substituiert können die Reste R⁵, R⁶, R⁷, R⁸ z. B. sein mit einem oder mehreren Resten, ausgewählt aus aliphatischen, alicyclischen, aromatischen, heteroaromatischen, gemischt aliphatisch-alicyclischen, gemischt aliphatischaromatischen, heterocyclischen, gemischt aliphatisch-heterocyclischen Kohlenwasserstoffresten mit 1 bis 50 Kohlcnstolfiatomcn, F, Cl, Br, I, -CF₃, -(CH₂)ᵢ(CF₂)ⱼCF₃ mit i = 0 - 9 und j = 0 - 9, - SiR²¹₃, -Si(OR²¹)₃, -SiR²1(OR²¹)₂, -SiR²¹₂OR²¹, -OSiR²¹₃, -OSi(OR²¹)₃, -OSiR²¹(OR²¹)₂, - OSiR²¹₂OR²¹, -OR¹⁹, -COR¹⁹, -CO₂R¹⁹, -CO₂M, -SO₂R¹⁹, -SOR¹⁹, -SO₃R¹⁹, -SO₃M, -SO₂NR¹⁹R²⁰, -NR¹⁹R²⁰, oder -N=CR¹⁹R²⁰, wobei R¹⁹, R²⁰, R²¹ und M die oben genannte Bedeutung aufweisen können. Beim Rest **Xa** stehen die Reste R⁵ bis R⁸ bevorzugt für Wasserstoff, Alkoxygruppen, insbesondere Methoxygruppen oder tert.-Butyl-Gruppen. Bevorzugt sind die Reste R⁵ und R⁶ und die Reste R⁷ und R⁸ jeweils paarweise gleich. Besonders bevorzugt sind die Reste R⁵ und R⁶ Methoxygruppen und/oder die Reste R⁷ und R⁸ tert.-Butyl-Gruppen.

In dem erfindungsgemäßen Bisphosphit ist das X bevorzugt ein mit Resten R¹, R²', R^{3'} und R^{4'} substituierter Ethylenrest, wobei die Reste R¹, R^{2'}, R^{3'} und R^{4'} gleiche oder verschiedene, substituierte oder unsubstituierte, verknüpfte, unverknüpfte oder kondensierte Aryl- oder Heteroarylreste sein können. Mögliche Substituenten für die Reste R^{1'} bis R^{4'} sind die für die Reste R¹ bis R⁴ genannten Substituenten. Besonders bevorzugte Bisphosphite sind solche, die symmetrisch sind, d. h. solche, bei denen X ein mit den Resten R¹, R^{2'} , R^{3'} und R^{4'} substituierter Ethylenrest ist, wobei die Reste R¹ und R¹, R² und R^{2'}, R³ und R³ und R⁴ und R⁴ jeweils gleich sind.

Der zweiwertige Rest Y im erfindungsgemäßen Bisphosphit kann vorzugsweise ein substituierter oder unsubstituierter Bisphenylrest oder Bisnaphthylrest sein. Mögliche Substituenten können die oben genannten Substituenten sein. Bevorzugt ist der Rest Y ausgewählt aus den Bisphenoxyresten der Formeln **IIa** bis **IId** 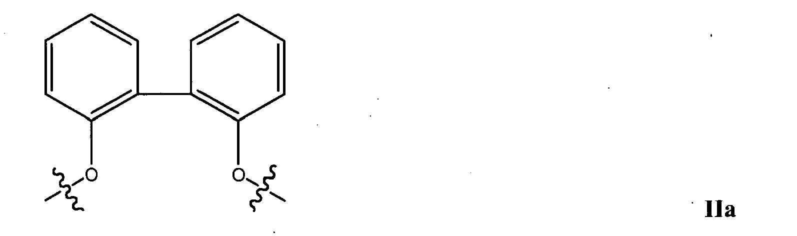 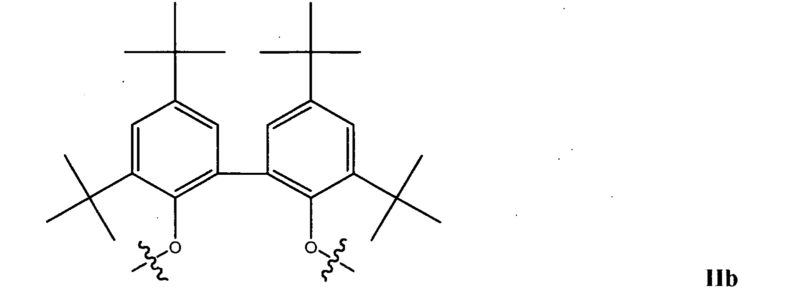 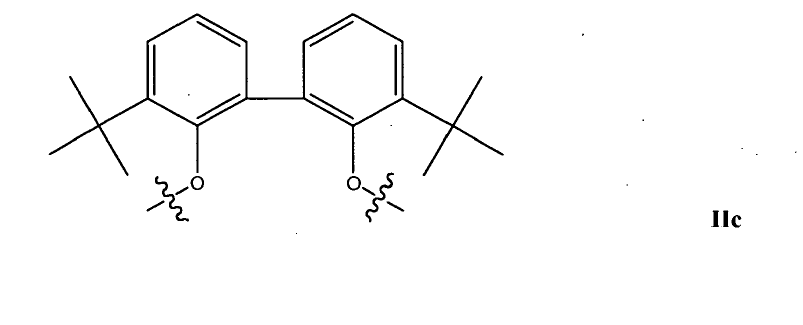 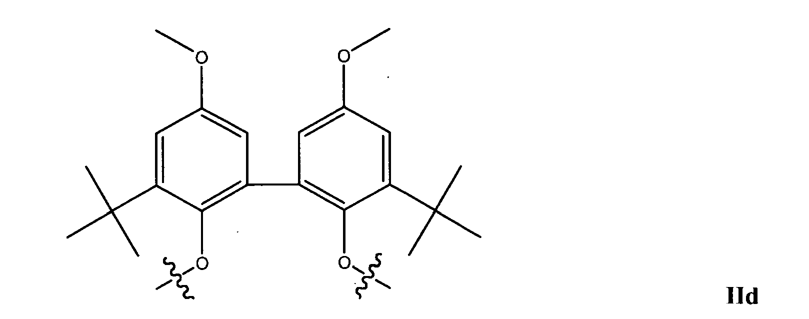 oder Bisnaphthoxyresten der Formel **III** 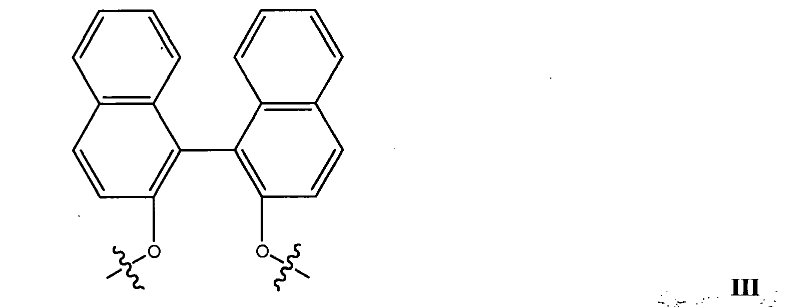 die in racemischer, atropisomerenangereicherter oder atropisomerenreiner Form vorliegen können.

Besonders bevorzugte erfindungsgemäße Bisphosphite sind die Bisphosphite der nachfolgenden Formeln **Ia** bis **Ic**, wobei **Ic** als Racemat oder in atropisomerenangereicherter oder atropisomerenreiner Form dargestellt und eingesetzt werden kann.

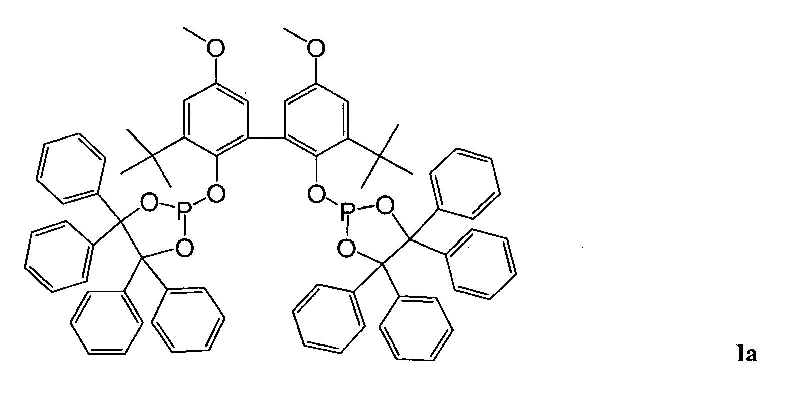

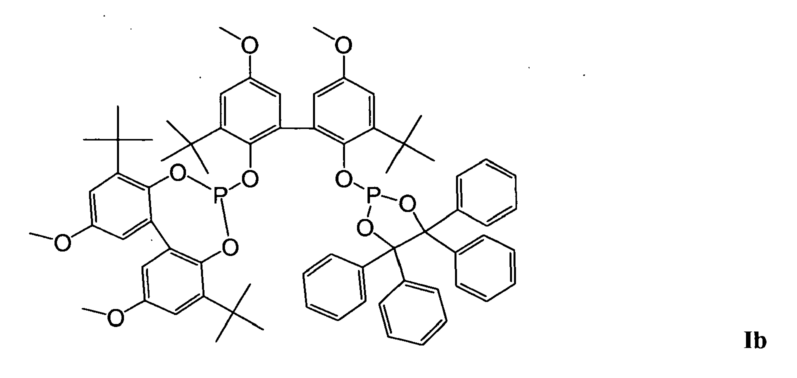

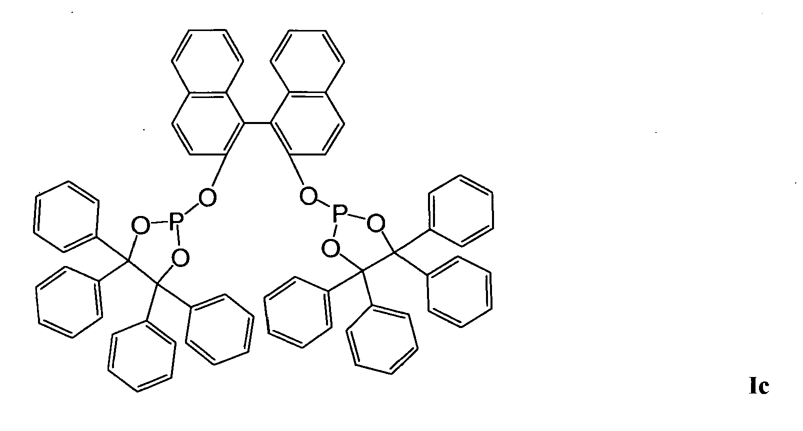

Die erfindungsgemäßen Bisphosphite zeichnen sich durch eine hohe Hydrolysestabilität aus und sind deshalb besonders geeignet, als Liganden eingesetzt zu werden. Durch den Einsatz von enantiomerenreinen Bisphosphiten kann die Hydroformylierung als asymmetrische Hydroformylierung durchgeführt werden.

Die erfindungsgemäßen Bisphosphite sind geeignete Liganden zur Komplexierung von Metallen der 4., 5., 6., 7., 8., 9. oder 10. Gruppe des Periodensystems der Elemente. Ein erfindungsgcmäßer Phosphitmetallkomplex zeichnet sich demgemäß dadurch aus, dass er ein Metall der 4., 5., 6., 7., 8., 9. oder 10. Gruppe des Periodensystems der Elemente und ein oder mehrere erfindungsgemäße Bisphosphite enthält. Die Komplexe können ein oder mehrere Phosphitliganden und gegebenenfalls weitere Liganden enthalten. Beispiele für geeignete Metalle sind Rhodium, Kobalt, Iridium, Nickel, Palladium, Platin, Eisen, Ruthenium, Osmium, Chrom, Molybdän und Wolfram. Vorzugsweise ist das Metall in dem erfindungsgemäßen Phosphitmetallkomplex Rhodium, Palladium, Nickel, Platin, Kobalt oder Ruthenium.

Die erfindungsgemäßen Bisphosphite oder die erfindungsgemäßen Metallkomplexe können in der Katalyse, insbesondere in der homogenen Katalyse verwendet werden. Insbesondere mit Metallen der 8., 9. oder 10. Gruppe können die resultierenden Metallkomplexe als Katalysatoren für Hydroformylierungs-, Carbonylierungs-, Hydrierungs- und Hydrocyanierungsreaktionen verwendet werden, besonders bevorzugt sind Rhodium, Kobalt, Nickel, Palladium, Platin und Ruthenium.

Besonders vorteilhaft kann es sein, wenn die erfindungsgemäßen Bisphosphite oder die erfindungsgemäßen Metallkomplexe in der katalytischen Hydroformylierung von Olefinen, vorzugsweise von Olefinen mit 2 bis 50 Kohlenstoffatomen, bevorzugt mit 3 bis 25, besonders bevorzugt mit 6 bis 12 und ganz besonders bevorzugt mit 8, 9, 10, 11 oder 12 Kohlenstoffatomen, verwendet werden. Bevorzugt wird ein Metallkomplex, der als Metall Rhodium oder Kobalt aufweist, in der Hydroformylierung eingesetzt. Das molare Verhältnis von Metall, bevorzugt einem Metall der 8. Gruppe, zu Bisphosphit in der Reaktionsmischung der Hydroformylierung beträgt vorzugsweise von 1 zu 1 bis 1 zu 500, bevorzugt von 1 zu 1 bis 1 zu 200 und besonders bevorzugt von 1 zu 2 bis 1 zu 50. Durch ein molares Verhältnis von Metall zu Bisphosphit von 1 zu größer 1, besonders bevorzugt von 1 zu größer 2 kann erreicht werden, dass das erfindungsgemäße Bisphosphit als freier Ligand in dem Reaktionsgemisch vorhanden ist.

Bei Einsatz von Rhodium als Katalysatormetall können sich in Hydroformylierungsreaktionen besonders hohe katalytische Aktivitäten ergeben. Katalysatoren, die Rhodium und erfindungsgemäßes Bisphosphit enthalten, zeichnen sich durch eine erstaunlich geringe Isomerisierungsaktivität sowie eine hohe Präferenz für endständige Doppelbindungen und einen hohen Anteil linearer Produktbildung aus und sind daher insbesondere zur selektiven terminalen Hydroformylierung endständiger Olefine geeignet. Sie können auch dazu verwendet werden, aus Olefingemischen oder Verbindungen mit end- und innenständigen Doppelbindungen gezielt die endständigen Olefine zu hydroformylieren, wobei die innenständigen Doppelbindungen weitgehend unangetastet bleiben.

Die Katalysatormetalle können in Form von Salzen oder Komplexen zum Einsatz kommen, im Falle von Rhodium z.B. als Rhodiumcarbonyle, Rhodiumnitrat, Rhodiumchlorid, Rh(CO)₂(acac) (acac = Acetylacetonat), Rhodiumacetat oder Rhodiumcarboxylate, beispielsweise Rhodiumoctanoat.

Aus den erfindungsgemäßen Bisphosphitliganden und dem Katalysatormetall bildet sich unter Reaktionsbedingungen die aktive Katalysatorspezies für die homogene Katalyse. Bei der Hydroformylierung bildet sich bei Kontakt des erfindungsgemäßen Bisphosphitliganden und des Katalysatormetalls mit Synthesegas vermutlich ein Carbonylhydridphosphit-Komplex als aktive Katalysatorspezies. Die Bisphosphite und gegebenenfalls weitere Liganden können in freier Form zusammen mit dem Katalysatormetall (als Salz oder Komplex) in die Reaktionsmischung gegeben werden, um die aktive Katalysatorspezies in situ zu erzeugen. Es ist weiterhin auch möglich, einen erfindungsgemäßen Phosphitmetallkomplex, der die o. g. Bisphosphitliganden und das Katalysatormetall enthält, als Vorstufe für den eigentlichen katalytisch aktiven Komplex einzusetzen. Diese Phosphitmetallkomplexe werden hergestellt, indem das entsprechende Katalysatormetall der 4. bis 10. Gruppe in Form einer chemischen Verbindung oder in der Oxidationsstufe 0 mit dem erfindungsgemäßen Bisphosphitliganden umgesetzt wird.

Frisches erfindungsgemäßes Bisphosphit kann zu jedem Zeitpunkt der Reaktion zugesetzt werden, um z. B. die Konzentration an freiem Liganden konstant zu halten.

Die Konzentration des Metalls im Reaktionsgemisch, insbesondere im Hydroformylierungsgemisch beträgt vorzugsweise von 1 Massen-ppm bis 1000 Massen-ppm und bevorzugt 5 Massen-ppm bis 300 Massen-ppm, bezogen auf das Gesamtgewicht der Reaktionsmischung.

Die mit den erfindungsgemäßen Bisphosphiten bzw. den entsprechenden Metallkomplexen durchgeführten Hydroformylierungsreaktionen können nach bekannten Vorschriften, wie z. B. in J. FALBE, "New Syntheses with Carbon Monoxide", Springer Verlag, Berlin, Heidelberg, New York, Seite 95 ff., (1980) beschrieben, durchgeführt werden. Die Olefinverbindungen werden dabei in Gegenwart des Katalysators mit einem Gemisch aus CQ und H₂ (Synthesegas) zu den um ein C-Atom reicheren Aldehyden umgesetzt.

Die Reaktionstemperaturen für ein Hydroformylierungsverfahren, bei dem die erfindungsgemäßen Bisphosphite bzw. Phosphitmetallkomplexe eingesetzt werden können, betragen vorzugsweise von 40 °C bis 180 °C, bevorzugt von 75 °C bis 140°C und besonders bevorzugt von 90 bis 120 °C. Der Druck, unter dem die Hydroformylierung durchgeführt wird, beträgt vorzugsweise von 1 bis 300 bar und bevorzugt von 10 bis 64 bar Synthesegas. Das Molverhältnis zwischen Wasserstoff und Kohlenmonoxid (H₂CO) im Synthesegas beträgt vorzugsweise 10/1 bis I/10 und besonders bevorzugt 1/1 bis 2/1.

Der Katalysator bzw. der Ligand/das erfindungsgemäße Bisphosphit ist vorzugsweise homogen im Hydroformylierungsgemisch, bestehend aus Edukten (Olefinen und Synthesegas) und Produkten (Aldehyden, Alkoholen, im Prozess gebildete Nebenprodukte, insbesondere Hochsieder), gelöst. Optional können zusätzlich Lösungsmittel oder Lösungsmittelgemische und/oder Stabilisatorverbindungen oder Promotoren verwendet werden.

Aufgrund ihres relativ hohen Molekulargewichtes besitzen die erfindungsgemäßen Bisphosphite eine geringe Flüchtigkeit. Sie können daher einfach von den leichter flüchtigen Reaktionsprodukten abgetrennt werden. Sie sind in den gängigen organischen Solventien ausreichend gut löslich.

Die Edukte für die Hydroformylierung können organische Verbindungen, insbesondere Olefine oder Gemische von Olefinen, vorzugsweise Olefine mit 2 bis 50, bevorzugt mit 3 bis 25 Kohlenstoffatomen insbesondere mit ein oder mehreren end- oder innenständigen C=C-Doppelbindungen sein. Sie können geradkettig, verzweigt oder von cyclischer Struktur sein. Beispiele sind Propen, 1-Buten, cis-2-Buten, trans-2-Buten, Isobuten, Butadien, Mischungen der C₄-Olefine, C₅-Olefine wie 1-Penten, 2-Penten, 2-Methylbuten-1, 2-Methylbuten-2, 3-Methylbuten-1, C₆-Olefine wie 1-Hexen, 2-Hexen, 3-Hexen, das bei der Dimerisierung von Propen anfallende C₆-Olefingemisch (Dipropen); C₇-Olefine wie 1-Hepten, weitere n-Heptene, 2-Methyl-1-hexen, 3-Methyl-1-hexen; C₈-Olefine wie 1-Octen, weitere n-Octene, 2-Methylheptene, 3-Methylheptene, 5-Methylhepten-2, 6-Methylhepten-2, 2-Ethylhexen-1, das bei der Dimerisierung von Butenen anfallende isomere C₈-Olefingemisch (Dibuten), C₉-Olefinc wie 1-Nonen, weitere n-Nonene, 2-Methyloctene, 3-Methyloctene, das bei der Trimerisierung von Propen anfallende C₉-Olefingemisch (Tripropen), C₁₀-Olefine wie n-Decene, 2-Ethyl-1-octen; C₁₂-Olefine wie n-Dodecene, das bei der Tetramerisierung von Propen oder der Trimerisierung von Butenen anfallende C₁₂-Olefingemisch (Tetrapropen oder Tributen), C₁₄-Olefine wie n-Tetradecene, C₁₆-Olefine wie n-Hexadecene, das bei der Tetramerisierung von Butenen anfallende C₁₆-Olefingemisch (Tetrabuten) sowie durch Cooligomerisierung von Olefinen mit unterschiedlicher C-Zahl (bevorzugt 2 bis 4) hergestellte Olefingemische, gegebenenfalls nach destillativer Trennung in Fraktionen mit gleicher oder ähnlicher C-Zahl. Ebenfalls können Olefine oder Olefingemische, die durch Fischer-Tropsch-Synthese erzeugt werden, eingesetzt werden, sowie Olefine bzw. C-C-Doppelbindungen aufweisende organische Verbindungen, die durch Oligomerisierung von Ethen erhalten werden oder die über Metathesereaktionen oder Telomerisationsreaktion zugänglich sind, wie z. B. Methylocta-2,7-dienylether.

Bevorzugte Edukte sind allgemein α-Olefine, wie Propen, 1-Buten, 1-Hexen oder 1-Octen, Dimere oder Trimere des Butens (Dibuten, Di-n-buten, Di-iso-buten, Tributen) sowie Methylocta-2,7-dienylether.

Es kann vorteilhaft sein, wenn im Hydroformylierungs-Reaktionsgemisch Verbindungen mit stabilisierenden Eigenschaften, insbesondere mit stabilisierenden Eigenschaften bezüglich der Stabilität der Liganden oder des Komplex-Katalysators vorhanden sind. Solche als Stabilisator einsetzbare Verbindungen können z. B.sterisch gehinderte Amine, wie sie z. B. in WO 2005/039762 beschrieben werden, insbesondere sterisch gehinderte sekundäre Amine, wie sie z. B. in DE 10 2005 042 464 beschrieben werden, sein.

Die Hydroformylierung kann kontinuierlich oder diskontinuierlich durchgeführt werden. Beispiele für technische Ausführungen sind Rührkessel, Blasensäulen, Strahldüsenreaktoren, Rohrreaktoren, oder Schlaufenreaktoren, die zum Teil kaskadiert und/oder mit Einbauten versehen sein können.

Die Reaktion kann durchgehend oder in mehreren Stufen erfolgen. Die Trennung der entstandenen Aldehydverbindungen und des Katalysators kann durch eine herkömmliche Methode durchgeführt werden. Technisch kann dies beispielsweise über eine Destillation, über einen Fallfilmverdampfer oder einen Dünnschichtverdampfer erfolgen. Dies gilt besonders, wenn der Katalysator in einem hochsiedenden Lösungsmittel gelöst von den niedriger siedenden Produkten abgetrennt wird. Die abgetrennte Katalysatorlösung kann für weitere Hydroformylierungen verwendet werden. Bei Einsatz niederer Olefine (z. B. Propen, Buten, Penten) ist auch ein Austrag der Produkte aus dem Reaktor über die Gasphase möglich.

Die erfindungsgemäßen Bisphosphite können durch eine Folge von Reaktionen von Phosphorhalogeniden mit Alkoholen, bei denen Halogenatome am Phosphor durch Sauerstoffgruppen substituiert werden, hergestellt werden. Eine geeignete Syntheseroute wird im Folgenden beschrieben.

So kann das erfindungsgemäße Bisphosphit z. B. durch ein Verfahren erhalten werden, welches die nachfolgenden Schritte
1) Umsetzen eines Diols **Aa** oder eines Alkoholamins **Ab** mit Phosphortrihalogenid zu Zwischenprodukt **A**,
2) Umsetzen eines Tetraarylethandiols **Ca**, das gleiche oder unterschiedliche, substituierte oder unsubstituierte, verknüpfte oder unverknüpfte, kondensierte oder unkondensierte Aryl- oder Heteroarylreste aufweisen kann, mit Phosphortrihalogenid zu Zwischenprodukt **C**,
3) Umsetzen des Zwischenprodukts **A** oder **C** mit einem Diol **Ba** zu einem Zwischenprodukt **B** und
4) Umsetzen des Zwischenprodukts **B** mit dem Zwischenprodukt **A** oder **C**, welches in Verfahrenschritt 3) nicht eingesetzt wurde,
umfasst.

Das Umsetzen der Diole mit dem Phosphortrihalogenid kann direkt erfolgen oder aber einen Zwischenschritt beinhalten. Es kann vorteilhaft sein, wenn die Diole **Aa**, **Ba**, **Ca**, das Alkoholamin **Ab** und/oder das Zwischenprodukt **C** in den Schritten 1 ), 2), 3) und/oder 4) mit einer Metallverbindung zum entsprechenden Metallsalz umgesetzt werden, welches dann mit dem Phosphortrihalogenid umgesetzt wird. Auf diese Weise entstehen bei der Umsetzung keine Hydrohalogenid-Verunreinigungen, insbesondere verhältnismäßig schwer abtrennbare Aminhydrochlorid-Verunreinigungen sondern es bilden sich Salze, die im Reaktionsmedium relativ schwer löslich sind und damit leicht, z. B. durch Filtration, abgetrennt werden können. Zudem ermöglicht dieser Zwischenschritt den Einsatz von nukleophilen Basen. Als Metallverbindung kann z. B. Natriumhydrid, Methyllithium oder Butyllithium eingesetzt werden.

Als Phosphortrihalogenid können z. B. PCl₃, PBr₃ und Pl₃, eingesetzt werden. Vorzugsweise wird als Phosphortrihalogenid Phosphortrichlorid (PCl₃) eingesetzt.

Es kann vorteilhaft sein, wenn die Umsetzung mit Phosphortrihalogenid in Gegenwart einer Base durchgeführt wird. Durch den Einsatz einer geeigneten Base kann der bei der Umsetzung der Phosphorhalogenide entstehende Halogenwasserstoff abgefangen bzw. katalysiert werden. Vorzugsweise wird als Base ein tertiäres Amin eingesetzt. Als tertiäre Amine können beispielsweise Triethylamin, Pyridin oder N-Butyldimethylamin eingesetzt werden. Zum Abfangen des entstehenden Halogenwasserstoffs wird vorzugsweise zumindest soviel Base eingesetzt, dass der entstehende Halogenwasserstoff vollständig abgefangen werden kann.

Da die eingesetzten Diole und ihre Folgeprodukte häufig fest sind, werden die Umsetzungen vorzugsweise in einem Lösungsmittel durchgeführt. Als Lösungsmittel können z. B. nicht protische Lösungsmittel, die weder mit den Alkoholen noch mit den Phosphorverbindungen reagieren, verwendet werden. Geeignete Lösungsmittel sind beispielsweise Ether wie Diethylether, Tetrahydrofuran (THF) oder MTBE (Methyl-tert.-butylether) oder aromatische Kohlenwasserstoffe wie Toluol.

Die eingesetzten Lösungsmittel sollten vorzugsweise weitestgehend wasser- und sauerstofffrei sein. Bevorzugt werden Lösungsmittel mit einem Wassergehalt von 0 bis 500 Massen-ppm, besonders bevorzugt von 5 bis 250 Massen-ppm verwendet. Der Wassergehalt kann beispielsweise durch das Verfahren nach Karl Fischer bestimmt werden.

Eine gegebenenfalls notwendige Trocknung der eingesetzten Lösungsmittel kann durch Destillation des Lösemittels über einem geeigneten Trockenmittel oder durch Durchströmen des Lösemittels durch eine beispielsweise mit Molekularsieb 4 Å gefüllte Kartusche oder Säule geschehen.

Die Verfahrensschritte in dem erfindungsgemäßen Verfahren zur Herstellung der Bisphosphite werden vorzugsweise bei einer Temperatur von -80°C bis 150 °C, bevorzugt von -20 °C bis 110 °C und besonders bevorzugt von 0 °C bis 80 °C durchgeführt.

Nachfolgend werden beispielhaft die vier Teilschritte des Verfahrens beschrieben. Bei dieser Ausführungsform des erfindungsgemäßen Verfahrens erfolgt zunächst die Umsetzung eines Diols **Aa** mit Phosphortrichlorid, zu Zwischenprodukt **A**.

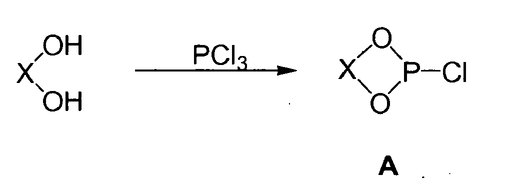

Ein Tetraarylethandiol **Ca**, das mit dem Diol aus dem vorangegangenen Schritt 1) identisch sein kann, aber nicht muss, wird mit Phosphortrichlorid zu Monohalogenphosphit **C** umgesetzt.

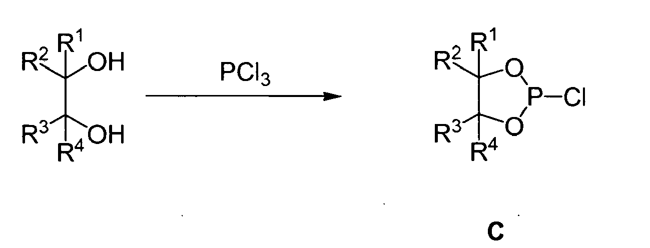

Das Zwischenprodukt **A** wird mit einem Diol **Ba** zu Monophosphit **B** umgesetzt.

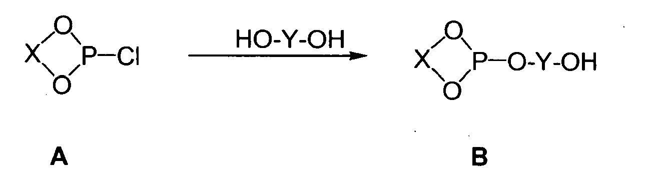

Zwischenprodukt **B** wird anschließend mit Zwischenprodukt **C** zum gewünschten Bisphosphit **D** umgesetzt.

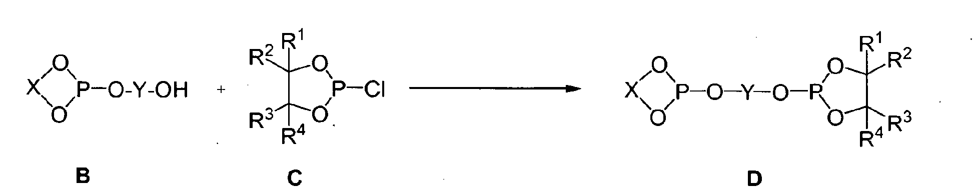

Dieser Syntheseweg ist nur einer von vielen und zeigt lediglich das grundsätzliche Vorgehen. Beispielsweise kann auch zunächst **C** mit HO-Y-OH (**Ba**)umgesetzt werden und anschließend **A** zugegeben werden. Bei symmetrischen Bisphosphiten, dass heißt, wenn X= R¹R²C-CR³R ist, können jeweils die Verfahrensschritte 1) und 2) sowie 3) und 4) zu einem Schritt zusammengefasst werden.

Als Ausgangsverbindung kann in dem erfindungsgemäßen Verfahren ein Diol **Aa**, oder ein Alkoholamin **Ab** eingesetzt werden.

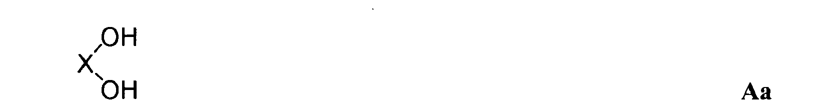

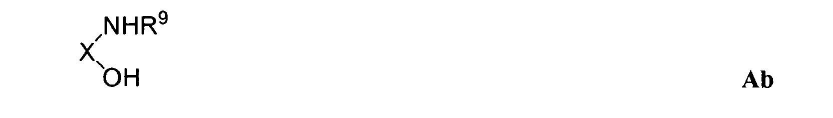

Für den Fall, dass die eingesetzte Verbindung ein Diol **Aa** ist, ist der Rest **X** vorzugsweise ein zweiwertiger substituierter oder unsubstituierter Bisalkylen- oder Bisarylenrest. Mögliche Substituenten der Bisalkylen- oder Bisarylenreste können die zu Formel **I** genannten Substituenten sein.

Vorzugsweise wird in dem erfindungsgemäßen Verfahren als Diol **Aa** eine Verbindung eingesetzt, bei der der Rest **X** insbesondere ein Rest **Xa** ist.

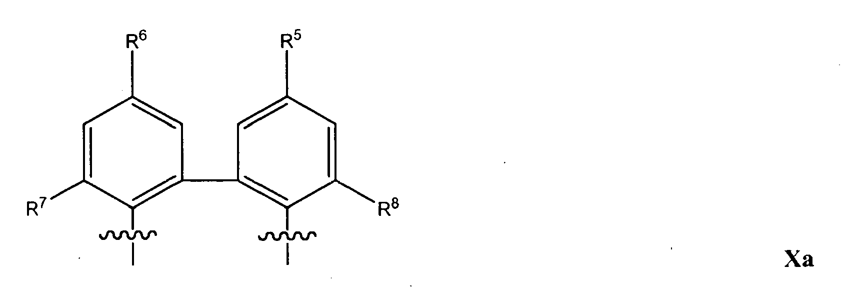

Die Reste R⁵, R⁶, R⁷, R⁸ können unabhängig voneinander substituierte oder unsubstituierte aliphatische, alicyclische, aromatische, heteroaromatische, gemischt aliphatisch-alicyclische, gemischt aliphatisch-aromatische, heterocyclische, gemischt aliphatisch-heterocyclische Kohlenwasserstoffreste mit 1 bis 50 Kohlenstoffatomen oder H, F, Cl, Br, I, -CF₃, -(CH₂)ᵢCF₂)ⱼCF₃ mit i = 0 - 9 und j = 0 - 9, -SiR²¹₃, -Si(OR²¹), -SiR²¹(OR²¹), -SiR²¹₂OR²¹, - OSiR²¹3, -OSi(OR²¹)₃, -OSiR²¹(OR²¹), -OSiR²¹2OR²¹, -OR¹⁹, -COR¹⁹, -CO₂R¹⁹ -CO₂M, -SO₂R¹⁹, -SOR¹⁹, -SO₃R¹⁹, -SO₃M, -SO₂NR¹⁹R²⁰, -NR¹⁹R²⁰, oder -N=CR¹⁹R²⁰, sein, wobei R¹⁹, R²⁰ und R²¹ unabhängig voneinander aus H, einwertigen substituierten oder unsubstituierten aliphatischen und aromatischen Kohlenwasserstoffresten mit 1 bis 25 Kohlenstoffatomen ausgewählt sind, wobei jedoch R²¹ = H ausgeschlossen ist und M ein Alkalimetall-, formal ein halbes Erdalkalimetall-, Ammonium- oder Phosphoniumion ist. Substituiert können die Reste R⁵, R⁶, R⁷, R⁸ z. B. sein mit mindestens einem Rest, ausgewählt aus aliphatischen, alicyclischen, aromatischen, heteroaromatischen, gemischt aliphatisch-alicyclischen, gemischt aliphatischaromatischen, heterocyclischen, gemischt aliphatisch-heterocyclischen Kohlenwasserstoffresten mit 1 bis 50 Kohlenstoffatomen, F, Cl, Br, I, -CF₃, -(CH₂)ᵢ(CF₂)ⱼCF₃ mit i = 0 - 9 und j = 0 - 9, - SiR²¹₃, -Si(OR²¹)₃, -SiR²¹(OR²¹)₂ -SiR²¹ ₂OR²¹, -OSiR²¹₃, -OSi(OR²¹)₃, -OSiR²¹(OR²¹)₂, - OSiR²¹₂OR²¹, -OR¹⁹, -COR¹⁹, -CO₂R¹⁹, -CO₂M, -SO₂R¹⁹, -SOR¹⁹, -SO₃R¹⁹, -SO₃M, -SO₂NR¹⁹R²⁰, -NR¹⁹R²⁰, oder -N=CR¹⁹R²⁰, wobei R¹⁹, R²⁰, R²¹ und M die oben genannte Bedeutung aufweisen können. Beim Rest **Xa** stehen die Reste R⁵ bis R⁸ bevorzugt für Wasserstoff, Alkoxygruppen, insbesondere Methoxygruppen oder tert.-Butyl-Gruppen. Bevorzugt sind die Reste R⁵ und R⁶ und die Reste R⁷ und R⁸ jeweils paarweise gleich. Besonders bevorzugt sind die Reste R³ und R⁶ Methoxygruppen und/oder die Reste R⁷ und R⁸ tert.-Butyl-Gruppen.

Es kann vorteilhaft sein, wenn ein Diol **Aa** eingesetzt wird, bei dem das X bevorzugt ein mit Resten R¹, R^{2'}, R^{3'} und R^{4'} substituierter Ethylenrest ist, wobei die Reste R¹ , R², R³ und R⁴ gleich oder verschieden substituierte oder unsubstituierte Arylreste sein können. Mögliche Substituenten für die Reste R¹ bis R⁴' sind die für die Reste R¹ bis R⁴ genannten Substituenten. Wird ein Diol **Ca** mit den Resten R¹ bis R⁴ und ein Diol **Aa** eingesetzt, bei dem X ein mit den Resten R¹, R², R^{3'} und R^{4'} substituierter Ethylenrest ist, und sind die Reste R¹ und R^{1'}, R² und R², R³ und R³ und R⁴ und R⁴ jeweils gleich, so kann ein symmetrisches erfindungsgemäßes Bisphosphit, wie z. B. eine Verbindung der Formeln **Ia** oder **Ic** hergestellt werden. Wie bereits weiter vorne ausgeführt, können, wenn die Ausgangsverbindungen **Aa** und **Ca** identisch sind, jeweils die Verfahrenschritte 1) und 2) bzw. 3) und 4) zusammengelegt bzw. gemeinsam durchgeführt werden.

Als Diol HO-Y-OH (**Ba**) können insbesondere solche eingesetzt werden, bei denen der zweiwertige Rest Y ein substituierter oder unsubstituierter Bisarylen- oder Bisalkylenrest, vorzugsweise ein Bisphenoxy- oder Bisnaphthoxyrest ist. Mögliche Substituenten können die zu Formel **I** genannten Substituenten sein. Bevorzugt wird ein Diol **Ba** eingesetzt, dessen Rest Y ausgewählt ist aus den Bisphenylresten der Formeln **IIa** bis **IId** 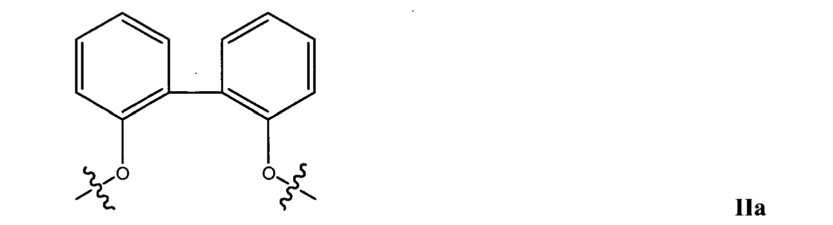 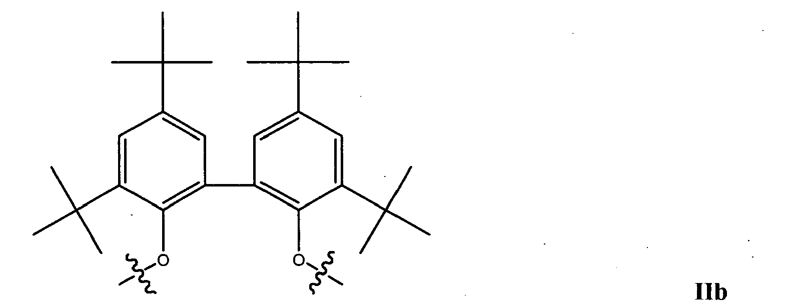 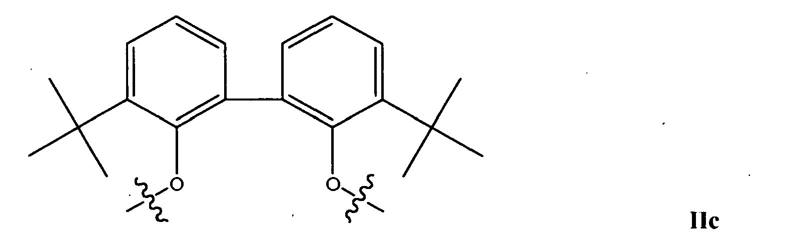 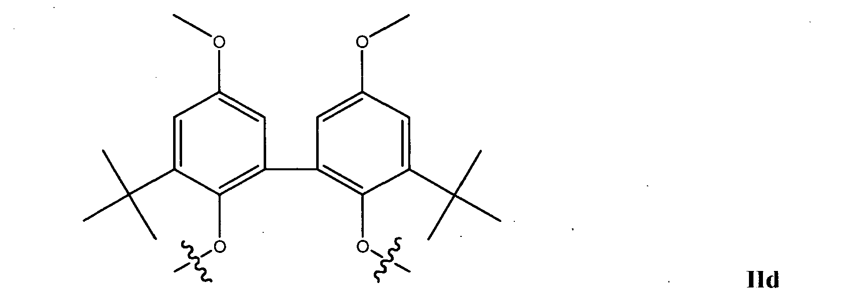 oder Bisnaphthylresten der Formel III in racemischer, enantiomerenangereicherter oder enantiomerenreiner Form

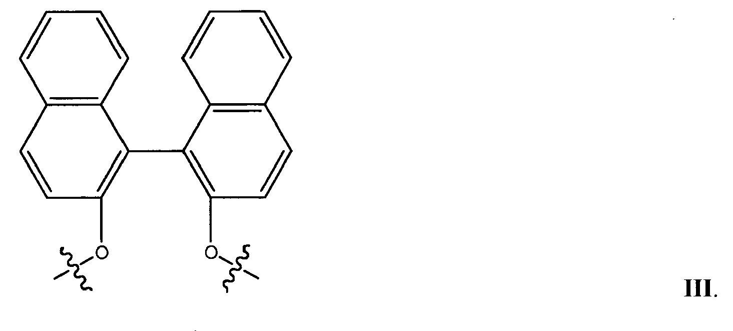

Als Tetraarylethandiol Ca 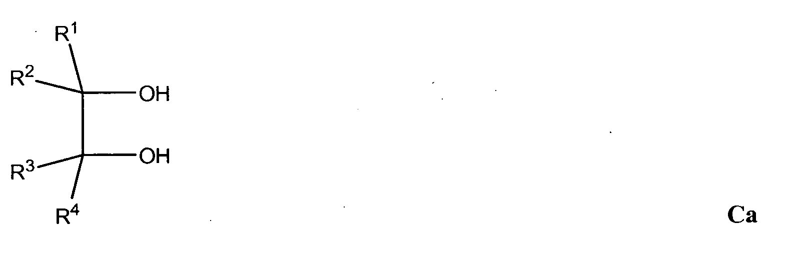 können Tetraarylethandiole eingesetzt werden, die substituierte oder unsubstituierte, verknüpfte, unverknüpfte oder kondensierte Aryl- oder Heteroarylreste R¹ bis R⁴ aufweisen. Die Reste R¹ bis R⁴ können gleich oder unterschiedlich sein. Mögliche Arylreste sind z. **B**. Phenylreste, Naphthylreste. Die Reste R¹ bis R⁴ können auch untereinander verknüpft oder kondensiert, wie z. B. 9-Fluorenyl-reste, Phenanthryl-Reste, Xanthenyl-Reste sowie Heteroaromaten sein.

Vorzugsweise wird als Tetraarylethandiol **Ca** Benzopinakol oder an den Phenylresten substituiertes Benzopinakol eingesetzt. Mögliche Substituenten der Reste R¹ bis R⁴ bzw. des Benzopinakol sind die zu Formel **I** genannten Substituenten.

Die folgenden Beispiele sollen das erfindungsgemäße Verfahren erläutern, ohne die Anwendungsbreite einzuschränken, die sich aus der Beschreibung und den Patentansprüchen ergibt.

### Beispiele

Alle Präparationen wurden unter Verwendung von Argon (Argon 5.0, Linde AG) als Schutzgas mittels Standard-Schlenkgefäß-Technik durchgeführt. Tetrahydrofuran und Toluol wurden vor Gebrauch über Kalium (THF) bzw. Natrium (Toluol) getrocknet. NMR-Spektren wurden mit einem Bruker ARX 400, Elementaranalysen mit einem LECO CHNS 932 durchgeführt.

### Beispiel 1: Synthese von 1-Chlor-3,3,4,4-tetraphenylphospholan (C-1)

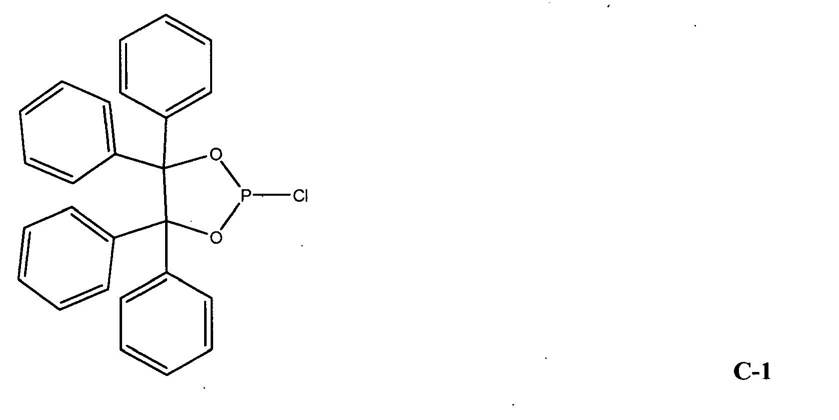

Eine Suspension von 1,878 g (5,12 mmol) Benzopinakol (Acros, 98 %) in 30 mL Tetrahydrofuran (Sigma-Aldrich, 99,9 %) wurde bei -40 °C unter Rühren tropfenweise mit einer 0,768 molaren Lösung von Phosphortrichlorid (Aldrich, 99 %) in Tetrahydrofuran (10,7 mL; 8,22 mmol) und anschließend tropfenweise mit einer Lösung von 1,56 g (15,4 mmol) Triethylamin (Aldrich, p.a.) in 4 mL Tetrahydrofuran versetzt, wobei sich ein voluminöser farbloser Niederschlag bildete. Man ließ das Gemisch auf Raumtemperatur erwärmen und rührte es für weitere 5 h. Die Reaktionsmischung wurde über eine G4-Fritte filtriert und das Filtrat bei 20 mbar 1,5 h bei 40 °C getrocknet. Der viskose Rückstand wurde in Toluol (15 mL) aufgenommen. Diese Lösung wurde über eine G4-Fritte filtriert, das Filtrat bei 20 mbar eingeengt und anschließend für 2 h bei 40 °C Badtemperatur bei 10⁻¹ mbar getrocknet. Es wurden 2,32 g (90 % der Theorie, berechnet als Toluol-Addukt) einer hochviskosen Flüssigkeit erhalten, die noch 0,8 Äquivalente Toluol enthält.
Analyse: ³¹P{1H}-NMR (C₆D₆) δ=173,44 ppm.

### Synthese von D-1

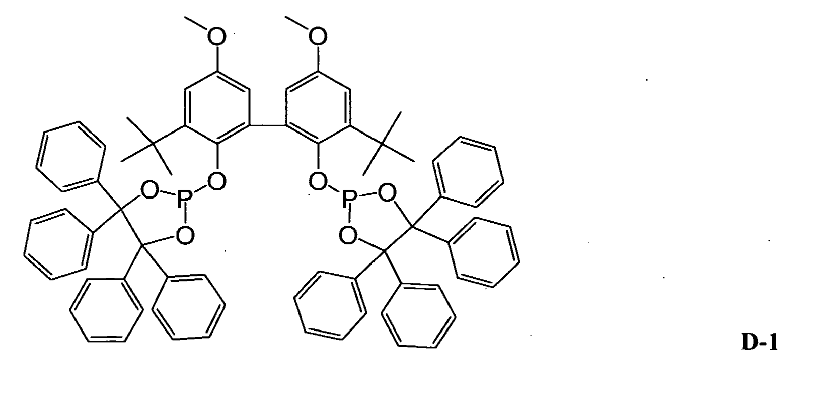

Zu 0,717 g (2 mmol) der Verbindung 2,2'-Bis(6-tert.-butyl-1-hydroxy-4-methoxyphenyl (hergestellt nach dem von F. R. Hewgill und D. G. Hewitt im Journal of the Chemical Society C, 1967, 8, 726-730 beschriebenen Verfahren) in Tetrahydrofuran (Riedel-de-Haën, Chromasolv) (8 mL) wurde bei -20 °C unter Rühren 4 mmol einer Lösung von n-Butyllithium (Aldrich) in 8 mL Hexan (Riedel-de-Haën, 97 %) getropft. Es wurde für 20 min bei -20 °C gerührt, anschließend auf Raumtemperatur erwärmt und danach tropfenweise eine Lösung von 1,90 g (4,4 mmol) von C-1 in Tetrahydrofuran (16 mL) zugegeben. Nach Rühren über Nacht bei 25 °C wurde das Lösungsmittel im bei 10⁻¹ mbar entfernt. Das erhaltene Produkt wurde mit 40 mL Toluol (Riedel-de-Haën, 99,7 %) verrührt, filtriert und der Frittenrückstand mit Toluol gewaschen. Die erhaltenen Filtrate wurden bei 10⁻¹ mbar aufkonzentriert und der Rückstand 2 h bei 40 °C getrocknet. Der erhaltene Feststoff wurde in 32 mL Acetonitril (Riedel-de-Haen, 99,9 %) aufgenommen und kurzzeitig bis zum Vorliegen einer klaren Lösung erhitzt. Beim Abkühlen fiel **D-1** aus. Es wurde abfiltriert und restliches Lösungsmittel bei 10⁻¹ mbar für 3 h bei 40 °C abgezogen und es wurden 1,9g (82 %) der Zielsubstanz erhalten.
³¹ P{¹H} -NMR (C₆D₆, 202 MHz): δ= 145,62 ppm.¹H-NMR (500 MHz, C₆D₆): δ= 1,39 (s, 18 H), 3,32 (s, 6 H), 6,84-6,88 (m, 7 H), 6,89-6,94 (m, 7 H), 6,95-7,00 (m, 4 H), 7,04-7,09 (m, 8 H), 7,21-7.30 (m, 10 H), 7.57 (d, J=7,5 Hz, 4 H), 7,65 (d, J=7,5 Hz, 4 H) ppm. Elementaranalyse (ber. für C₇₄H₆₈O₈P₂ = 1147,29 g/mol): C 77,00 (77,47); H 5,66 (5,97); P 5,82 (5,40) %.

### Beispiel 2: Synthese von A-1

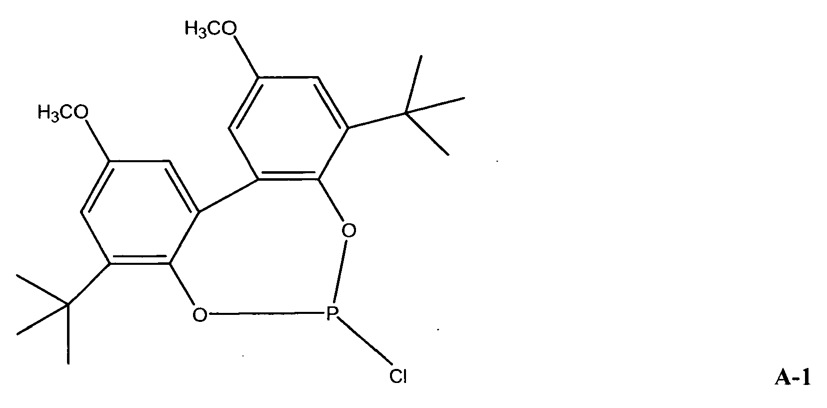

**A1** wurde aus 2,2'-Bis(6-tert.-butyl-1-hydroxy-4-methoxyphenyl) (hergestellt nach dem von F. R. Hewgill und D. G. Hewitt im Journal of the Chemical Society C, 1967, 8, 726-730 beschriebenen Verfahren) nach der in EP 1 209 164 angegebenen Methode hergestellt.

### Synthese von B-2

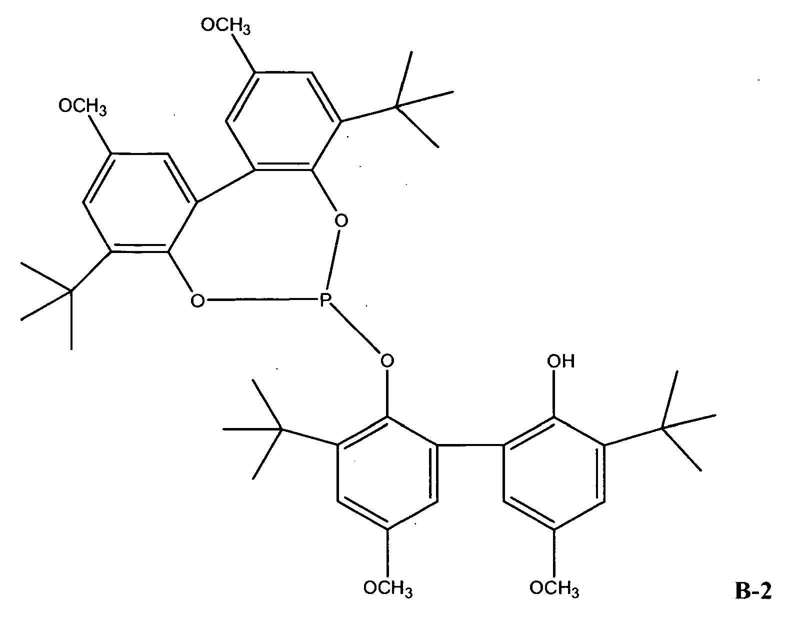

**B-2** wurde nach dem in EP 1 209 164 (Beispiel 2) beschriebenen Verfahren aus **A-1** hergestellt.

### Synthese von D-2

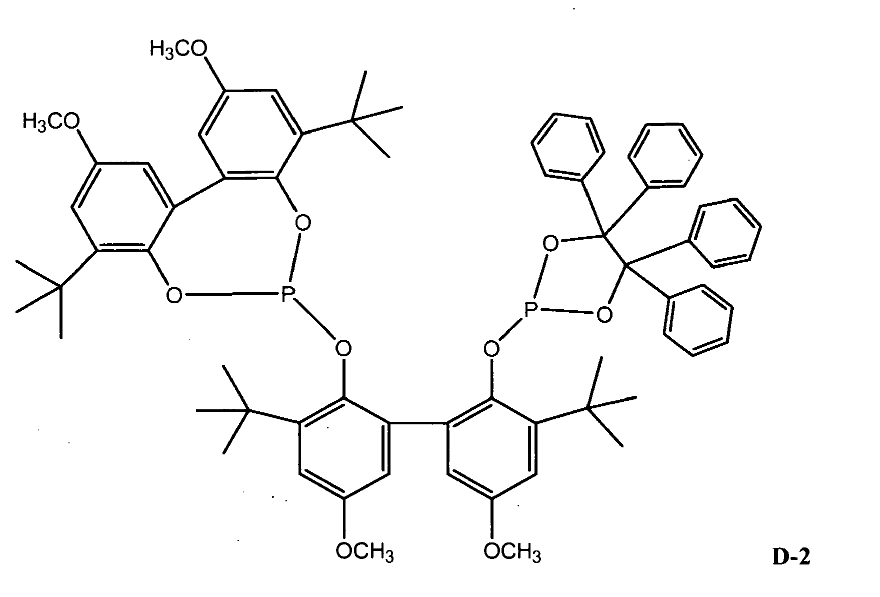

Zu einer auf -20 °C gekühlten Lösung von **B-2** (1,5 g; 2,014 mmol) in THF (8 ml) wurde unter Rühren n-Butyllithium (2,014 mmol) in Hexan (4 ml) getropft. 20 min nach beendeter Zugabe wurde die Mischung auf Raumtemperatur gebracht und langsam tropfenweise mit einer Lösung von 2-Chlor-4,4',5,5'-tetraphenyl-1,3,2-dioxaphospholan **(C-1)** (0,868 g; 2,014 mmol) in THF (8 ml) versetzt. Es wurde 4 h bei Raumtemperatur gerührt, danach das Lösungsmittel bei 40 mbar entfernt und der bei 40° bei 10⁻¹ mbar getrocknete Rückstand anschließend mit Toluol (30 ml) verrührt. Nach Filtration wurde das Filtrat eingeengt, und der erhaltene weiße Feststoff bei 10⁻¹ mbar getrocknet (40 °C, 2 h). Das Rohprodukt wurde mit 40 ml siedendem Acetonitril verrührt. Diese Mischung wurde nach Lagerung im Kühlschrank filtriert und der Filterkuchen mit eiskaltem Acetonitril gewaschen. Trocknung bei 10⁻¹ mbar ergab 1,6 g (1,404 mmol= 70 % d. Theorie) an Produkt. Analyse (ber. für C₇₀H₇₆O₁₀P₂ = 1139,31 g/ Mol) C 73,61 (73,80); H 6,99 (6,72); P 5,46 (5,44)%. ³¹P-NMR (CD₂Cl₂) δ 1.35,4 (d, Jpp= 38,9 Hz); 142,9 ppm (d, Jpp,= 38,9 Hz). ¹H-NMR (CD₂Cl₂) δ1,26; 1,28; 1,38; 1,46 (4x s, je 9 H); 3,48; 3,81; 3,82; 3,86 (4x s, je 3 H); 6,42 (m, 1 H); 6,69 (m, 1 H); 6,78 (m, 1 H); 6,94-7,10 (m, 18 H); 7,19-7,25 (m, 5 H); 7,61 (m, 2 H) ppm.

### Beispiel 3: Synthese von D-3

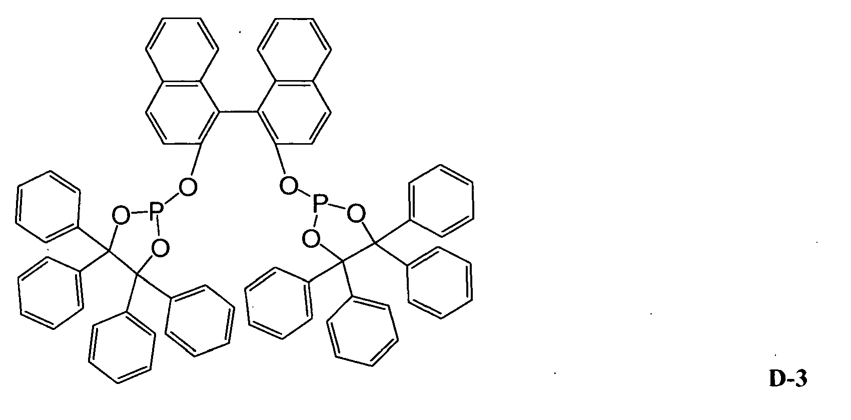

Zu einer Suspension von *rac*-1,1'-Bi(2-naphthol) (0,891 g, 3,113 mmol, Aldrich, 99 %) in 18 mL Toluol wurde zunächst bei Raumtemperatur Triethylamin (0,992 g; 9,81 mmol) gegeben und dann bei 0 °C unter Rühren eine Lösung von **C-1** (2,951 g; 6,85 mmol) in 26 ml Toluol gegeben. Es wurde noch 45 min bei 0 °C und dann 16 h bei 80 °C gerührt. Das erhaltene Produkt wurde filtriert, das Lösungsmittel bei 20 mbar entfernt und nach Verrühren des Rückstandes mit 10 mL Toluol erneut filtriert. Das Filtrat wurde eingeengt, der erhaltene hochviskose Rückstand 2 h bei 55 °C bei 10⁻¹ mbar getrocknet und anschließend aus Acetonitril umkristallisiert. Ausbeute: 2,684 g (2,496 mmol; 80 % d. Theorie). Analyse (ber. für C₇₂H₅₂O₆P₂ = 1075,14 g/ Mol) C 79,63 (80,43); H 4,69 (4,87); P 6,03 (5,76) %. ³¹P-NMR (CD₂Cl₂) δ 140,5 ppm.

### Beispiel 4: Vergleichsversuche zur Hydroformylierung mit Liganden D-1, D-2, D-3 und Vergleichsligand D-4

Als Vergleichsligand wurde das in EP 0 472 071 beschriebene Bisphosphit **D-4** gewählt. Die Synthese von **D-4** erfolgte analog EP 0 472 071. Alle Beispiele mit dem bekannten Liganden **D-4** dienen lediglich dem Vergleich und sind daher nicht erfindungsgemäß.

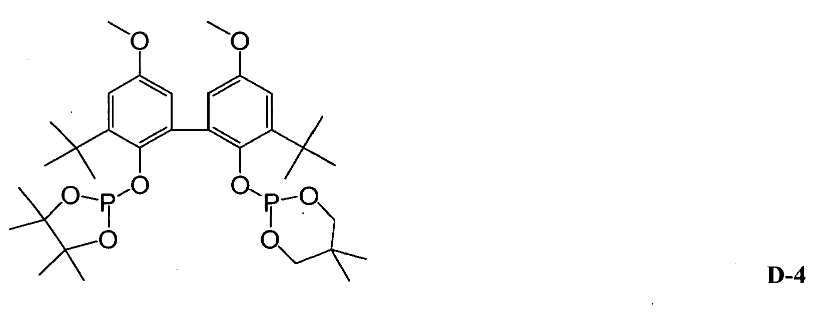

Die Hydroformylierung wurde in einem, mit Druckkonstanthaltung, Gasflussmessung, Begasungsrührer und Druckpipette ausgestatteten 200 ml-Autoklaven der Fa. Premex Reactor AG, Lengau, Schweiz, durchgerührt. Für die Versuche wurden im Autoklaven unter Argonatmosphäre folgende Lösungen des Rhodiums in Form von [(acac)Rh(COD)] (acac=Acetylacetonat-Anion; COD=1,5-Cyclooctadien) (OMG AG & Co. KG, Hanau, DE) als Katalysatorvorstufe in Toluol eingehüllt: Für Versuche mit 38,4 Massen-ppm Rhodium 10 ml einer 1,724 millimolaren Lösung und für Versuche mit 96 Massen-ppm Rhodium 10 ml einer 4,31 millimolaren Lösung. Anschließend wurde die entsprechende Menge der in Toluol gelösten Phosphitverbindung, in der Regel 5 Ligandäquivalente pro Rhodium, zugemischt. Durch Zugabe von weiterem Toluol wurde das Anfangsvolumen der Katalysatorlösung auf 41 ml eingestellt. In die Druckpipette gab man 15 ml des in Tabelle 1 jeweils angegebenen Olefins oder Olefingemischs, wobei zuvor dessen Masse bestimmt wurde. Nach Austausch der Argonatmosphäre durch Spülen mit Synthesegas (Linde, H₂ (99,999 %) : CO (99,997 %) = 1 : 1 ) wurde bei einem Synthesegasdruck von a) 33 bar für einen Enddruck von 50 bar, b) 12 bar für einen Enddruck von 20 bar und c) 7 bar für einen Enddruck von 10 bar unter Rühren (1500 U/min) auf die jeweils angegebenen Temperaturen aufgeheizt. Nach Erreichen der Reaktionstemperatur wurde der Synthesegasdruck auf a) 48 bar für einen Enddruck von 50 bar, b) 19 bar für einen Enddruck von 20 bar und c) 9,5 bar für einen Enddruck von 10 bar erhöht und das jeweils in Tabelle 1 angegebene Olefin(-gemisch) mit einem in der Druckpipette eingestellten Überdruck von ca. 3 bar zugepresst. Die Reaktion wurde bei konstantem Druck von jeweils 50 bzw. 20 bzw. 10 bar (Nachdruckregler der Fa. Bronkhorst, NL) über eine Reaktionszeit von jeweils 4 h durchgeführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurde unmittelbar nach Abschalten des Rührers entnommen, mit 5 ml Pentan verdünnt und gaschromatographisch analysiert.

GC-DATEN: HP 5890 Series II plus, PONA, 50 m x 0,2 mm x 0,5 µm; quantitative Bestimmung von Restolefin und Aldehyd gegen das Lösungsmittel Toluol als internen Standard.

Tabelle 1 gibt die Resultate der rhodiumkatalysierten Hydroformylierung von 1-Octen (Aldrich, 98 %), 2-Penten, Propen (ABCR, cis+trans, 99 %) und einem Isomerengemisch von Octenen, welches eine Zusammensetzung wie in DE 100 53 272, Beispiele 9 bis 19 beschrieben aufweist, wieder.

Zusätzlich gibt die Tabelle 1 die in EP 0 472 071 für die Hydroformylierung von 2-Buten angegebenen Ergebnisse wieder. Geringe Umsätze bei dem Octengemisch bzw. bei 2-Penten und 2-Buten belegen geringe Isomerisierungsaktivität, hohe Umsätze weisen auf erhebliche unerwünschte Isomerisierungsaktivität hin. Die Selektivität gibt den Anteil an endständig hydroformyliertem Substrat an.

**Tabelle 1: Hydroformylierung end- und innenständiger Olefine, Versuchsergebnisse**

| Ligand | Substrat | Rh [ppm] | Ligand/ Rhodium | Temp. [°C] | Solvens | Druck [bar] | Umsatz [%] | n-Selektivität [%] |
|---|---|---|---|---|---|---|---|---|
| **D-1** | 1-Octen | 38,4 | 5 | 120 | Toluol | 50 | 86 | 99 |
| **D-2** | 1-Octen | 38,4 | 5 | 100 | Toluol | 50 | 70 | 94 |
| **D-3** | 1-Octen | 38,4 | 5 | 100 | Toluol | 50 | 89 | 83 |
| **D-4** | 1-Octen | 40 | 5 | 100 | Toluol | 50 | 94 | 85 |
| **D-1** | n-Octene | 96 | 5 | 120 | Toluol | 20 | 8 | 99 |
| **D-2** | n-Octene | 96 | 5 | 120 | Toluol | 20 | 19 | 77 |
| **D-3** | n-Octene | 96 | 10 | 120 | Toluol | 10 | 7 | 89 |
| **D-4** | n-Octene | 96 | 5 | 120 | Toluol | 20 | 54 | 33 |
| **D-1** | 2-Penten | 96 | 5 | 120 | Toluol | 20 | 14 | 99 |
| **D-1** | 2-Penten | 96 | 2 | 120 | Toluol | 20 | 14 | 99 |
| **D-4** | 2-Penten | 96 | 5 | 120 | Toluol | 20 | 86 | 60 |
| **D-4** | 2-Penten | 96 | 2 | 120 | Toluol | 20 | 80 | 60 |
| **D-4^{a}** | 2-Buten | 90 | 4,8 | 90 | Hexanol® | 25 | 52 | 31 |
| **D-1^{c}** | Propen | 40 | 5 | 90 | Toluol | 20 | 84 | 97 |
| **D-4^{b}** | Propen | 90 | 4,1 | 70 | Texanol® | 30 | 81 | 91 |

| | | | | | | | | |
|---|---|---|---|---|---|---|---|---|
| ^{a} entnommen EP 0 472 071 (Beispiel 9, Tabelle 6, Eintrag 3). ^{b} entnommen EP 0 472 071 (Beispiel 3). ^{c} in Gegenwart von 0,0636g Tinuvin S (Ciba); Umsatz und Selektivität nach 5 h. | | | | | | | | |

Wie Tabelle 1 zeigt, zeigten alle Liganden ähnliche Aktivitäten und Selektivitäten bei der Hydroformylierung des endständigen Olefins, wobei **D-1** bislang unerreichte Selektivitäten erzielte. Deutliche Unterschiede zeigten sich jedoch beim Octengemisch sowie bei der Umsetzung von 2-Penten: Während **D1-D3** die innenständigen Doppelbindungen weitgehend unangetastet ließen, zeigte die Vergleichssubstanz **D-4** signifikante Isomerisierungsaktivität, was durch hohe Umsätze (86 % bei 2-Penten) belegt wird. Die geringen Selektivitäten bei **D-4** zeigen zudem, dass in großem Maße verzweigte Aldehyde gebildet wurden. Hingegen wurden bei der Verwendung von **D-1** die geringen Mengen (14 %) zu 1-Penten isomerisierten 2-Pentens mit 99 % Linearität hydroformyliert. Der Vergleich mit Propen als Substrat belegt zudem die überlegene n/iso-Selektivität des erfindungsgemäßen Liganden bzw. Katalysatorkomplexes.

### Beispiel 5: Hydroformylierung von Methylocta-2,7-dienylether (MODE)

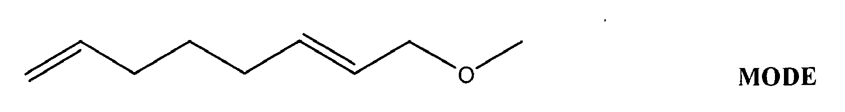

Methylocta-2,7-dienylether (MODE) ist eine Verbindung mit einer end- und einer innenständigen Doppelbindung. In diesem Beispiel sollte gezielt die endständige Doppelbindung hydroformyliert werden, die innenständige sollte möglichst unangetastet bleiben. Die Hydroformylierung wurde wie in Beispiel 4 beschrieben durchgeführt.

**Tabelle 2: Hydroformylierung von Mode, Parameter und Versuchsergebnisse**

| Ligand | Substrat | Rh [ppm] | Ligand/ Rhodium | Temp. [°C] | Solvens | Druck [bar] | Umsatz [%] | n-Selektivität [%] |
|---|---|---|---|---|---|---|---|---|
| **D-1** | MODE | 40 | 5 | 100 | Toluol | 50 | 82 | 95 |

Bei Einsatz der in EP 0 472 071 beschriebenen, nicht erfindungsgemäßen Vergleichsverbindung **D-4** wurde hingegen kein einheitliches Produkt erhalten. Man erhielt ein Produktgemisch, das etwa 50 Massen-% 9-Methoxynon-7-enal enthielt. Das erfindungsgemäße Phosphit ist also deutlich selektiver als die nicht erfindungsgemäße Vergleichssubstanz.

### Beispiel 6: Hydrolysestabilität:

0,05 mol der in Tabelle 3 jeweils angegebenen Verbindung wurden in jeweils 1 mL Toluol-d₈ (Fluka) gelöst und ein ³¹P-NMR-Spcktrum aufgenommen. Anschließend wurden 18 mg Wasser (1 mmol, 20 Äquivalente) hinzu gegeben und für 1 min kräftig geschüttelt. Man ließ die Mischung bei Raumtemperatur stehen und nahm nach zunächst 2 h, dann nach 6 Tagen ein ³¹P-NMR-Spektrum auf.

**Tabelle 3: Hydrolysetests**

| Ligand | Zeit [h] | 31P-NMR-spektroskopische Reinheit Ligand |
|---|---|---|
| **D-1** | 0 | > 99 % |
| **D-1** | 2 | > 99 % |
| **D-1** | 144 | > 99 % |
| **D-2** | 0 | > 95 % |
| **D-2** | 2 | > 95 % |
| **D-2** | 144 | > 95 % |
| **D-4** | 0 | > 99 % |
| **D-4** | 2 | < 5 % |
| **D-4** | 144 | < 1 %^{a} |

| | | |
|---|---|---|
| ^{a} Substanz nicht mehr detektierbar, vollständige Zersetzung | | |

Tabelle 3 zeigt, dass die erfindungsgemäßen Phosphite eine deutlich erhöhte Hydrolysestabilität zeigen als das nicht erfindungsgemäße Vergleichsphosphit D-4 aus EP 0 472 071.

## Patentansprüche

1. Bisphosphit der Formel I 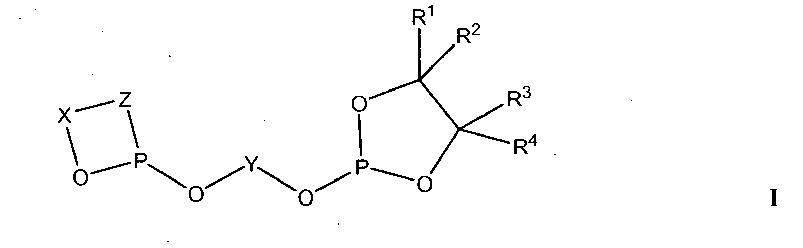 mit X = zweiwertiger substituierter oder unsubstituierter Bisalkylen- oder Bisarylenrest, der ein oder mehrere Heteroatom(e) enthalten kann, Y = zweiwertiger substituierter oder unsubstituierter Bisarylen- oder Bisalkylenrest, der ein oder mehrere Heteroatom(e) enthalten kann, Z = Sauerstoff oder NR⁹, R¹, R² , R³, R⁴ gleiche oder verschiedene, substituierte oder unsubstituierte, verknüpfte, unverknüpfte oder kondensierte Aryl- oder Heteroarylreste, und R⁹ = Wasserstoff oder substituierter oder unsubstituierter Alkyl- oder Arylrest, der ein oder mehrere Heteroatom(e) enthalten kann.

2. Bisphosphit nach Anspruch 1,
**dadurch gekennzeichnet,**
**dass** X ein Rest **Xa** ist, 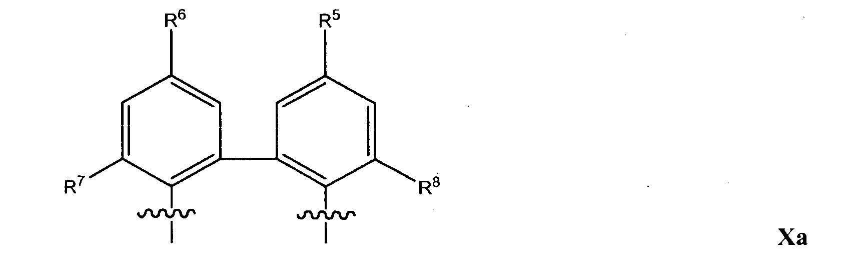 wobei die Reste R⁵, R⁶, R⁷, R⁸ unabhängig voneinander substituierte oder unsubstituierte aliphatische, alicyclische, aromatische, heteroaromatische, gemischt aliphatisch-alicyclische, gemischt aliphatisch-aromatische, heterocyclische, gemischt aliphatisch-heterocyclische Kohlcnwasscrstoffiestc mit 1 bis 50 Kohlenstoffatomen oder H, F, Cl, Br, I, -CF₃, -(CH₂)ᵢ(CF₂)ⱼCF₃ mit i = 0 - 9 und = 0 - 9, -SiR²¹3, -Si(OR²¹)₃, -SiR²¹(OR²¹)₂ -SiR²¹₂OR²¹, -OSiR²¹₃, -OSi(OR²¹)₃, -OSiR²¹(OR²¹)₂, -OSiR²¹₂OR²¹, -OR¹⁹, -COR¹⁹, -CO₂R¹⁹, -CO₂M, -SO₂R¹⁹, -SOR¹⁹, -SO₃R¹⁹, -SO₃M, -SO₂NR¹⁹R²⁰, -NR¹⁹R²⁰, oder -N=C^{R19} R²⁰, sind, wobei R¹⁹, R²⁰ und R²¹ unabhängig voneinander aus H, einwertigen substituierten oder unsubstituierten aliphatischen und aromatischen Kohlenwasserstoffresten mit 1 bis 25 Kohlenstoffatomen ausgewählt sind, wobei jedoch R²¹ = H ausgeschlossen ist und M ein Alkalimetall-, formal ein halbes Erdalkalimetall-, Ammonium- oder Phosphoniumion ist.

3. Bisphosphit nach Anspruch 1,
**dadurch gekennzeichnet,**
**dass** X ein mit den Resten R¹, R², R³ und R⁴ substituierter Ethylenrest ist.

4. Bisphosphit nach Anspruch 3,
**dadurch gekennzeichnet,**
**dass** X ein mit den Resten R¹, R², R³ und R⁴ substituierter Ethylenrest ist, wobei die Reste R¹ und R¹, R² und R^{2'}, R³ und R^{3'} und R⁴ und R^{4'} jeweils gleich sind.

5. Bisphosphit nach einem der Ansprüche 1 bis 4,
**dadurch gekennzeichnet,**
**dass** die Reste R¹, R², R³, R⁴ unsubstituierte Phenylreste sind.

6. Bisphosphit nach einem der Ansprüche 1 bis 5,
**dadurch gekennzeichnet,**
**dass** Y ausgewählt ist aus Bisphenoxyresten der Formel **IIa** bis **IId** 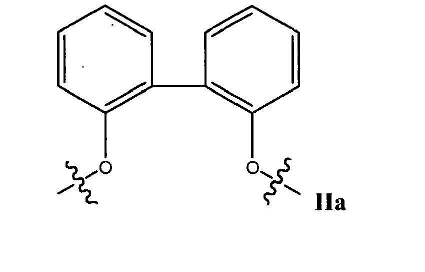 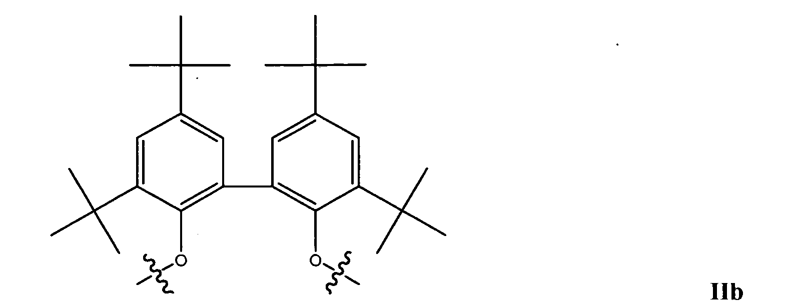 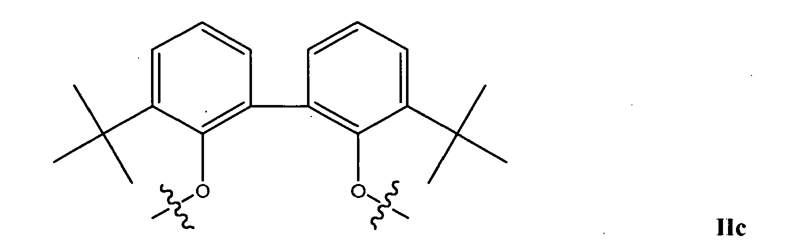 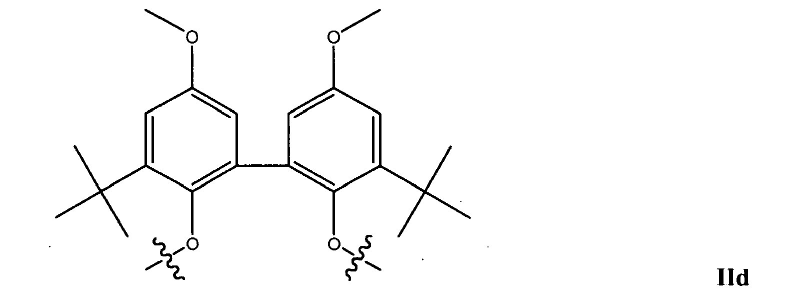 oder Bisnaphthoxyresten der Formel **III**

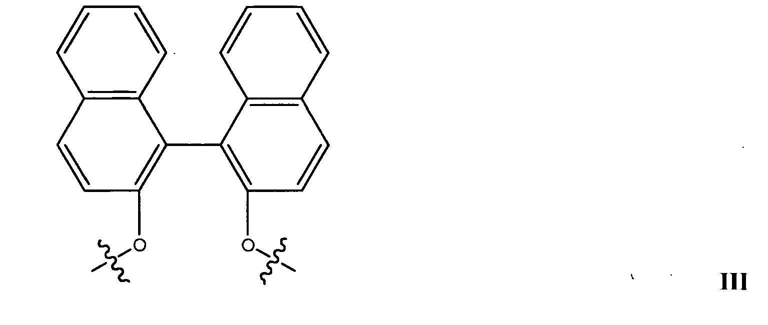

7. Phosphitmetallkomplex, enthaltend ein Metall der 4., 5., 6., 7., 8., 9. oder 10. Gruppe des Periodensystems der Elemente und ein oder mehrere Bisphosphite gemäß einem der Ansprüche 1 bis 6.

8. Phosphitmetallkomplex nach Anspruch 7,
**dadurch gekennzeichnet,**
**dass** das Metall Rhodium, Palladium, Nickel, Platin, Kobalt oder Ruthenium ist.

9. Verwendung eines Bisphosphits der Ansprüche 1 bis 6 oder eines Metallkomplexes gemäß Anspruch 7 oder 8 in der Katalyse.

10. Verwendung gemäß Anspruch 9,
**dadurch gekennzeichnet,**
**dass** die Katalyse eine homogene Katalyse ist.

11. Verwendung nach Anspruch 9 oder 10,
**dadurch gekennzeichnet,**
**dass** die katalysierte Reaktion die Hydroformylierung von Olefinen ist.

12. Verwendung nach 11,
**dadurch gekennzeichnet,**
**dass** ein Metallkomplex, der als Metall Rhodium oder Kobalt aufweist, in der Hydroformylierung eingesetzt wird.

13. Verwendung nach einem der Ansprüche 11 oder 12,
**dadurch gekennzeichnet,**
**dass** das molare Verhältnis von Metall zu Bisphosphit in der Reaktionsmischung von 1 zu 1 bis 1 zu 500 beträgt.

14. Verfahren zur Herstellung eines Bisphosphits gemäß einem der Ansprüche 1 bis 6,
**dadurch gekennzeichnet,**
**dass** es die nachfolgenden Schritte
1) Umsetzen eines Diols **Aa** oder eines Alkoholamins **Ab** mit Phosphortrihalogenid zu Zwischenprodukt **A**,
2) Umsetzen eines Tetraarylethandiols **Ca**, das gleiche oder unterschiedliche, substituierte oder unsubstituierte, verknüpfte oder unverknüpfte, kondensierte oder unkondensierte Aryl- oder Heteroarylreste aufweist, mit Phosphortrihalogenid zu Zwischenprodukt **C**,
3) Umsetzen des Zwischenprodukts **A** oder **C** mit einem Diol **Ba** zu einem Zwischenprodukt **B** und
4) Umsetzen des Zwischenprodukts **B** mit dem Zwischenprodukt **A** oder **C**, welches in Verfahrenschritt 3) nicht eingesetzt wurde.

15. Verfahren nach Anspruch 14,
**dadurch gekennzeichnet,**
**dass** die Diole **Aa**, **Ba**, **Ca** oder das Alkoholamin **Ab** oder und/oder das Zwischenprodukt **C** in den Schritten 1), 2), 3) und/oder 4) mit einer Metallverbindung zum entsprechenden Metallsalz umgesetzt wird, welches dann mit dem Phosphortrihalogenid umgesetzt wird.

16. Verfahren nach Anspruch 15,
**dadurch gekennzeichnet,**
**dass** als Metallverbindung Natriumhydrid, Methyllithium oder Butyllithium eingesetzt wird.

17. Verfahren nach zumindest einem der Ansprüche 14 bis 16,
**dadurch gekennzeichnet,**
**dass** die Umsetzung mit Phosphortrihalogenid in Gegenwart einer Base durchgeführt wird.

18. Verfahren nach Anspruch 17,
**dadurch gekennzeichnet,**
**dass** ein tertiäres Amin als Base eingesetzt wird.

## Claims

1. Bisphosphite of the formula **I** 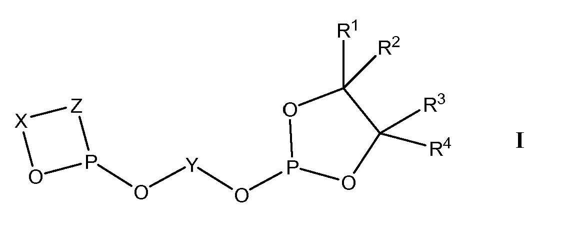 where X = a divalent substituted or unsubstituted bisalkylene or bisarylene radical which may contain one or more heteroatom(s), Y = a divalent substituted or unsubstituted bisarylene or bisalkylene radical which may contain one or more heteroatom(s), Z = oxygen or NR⁹, R¹, R², R³, R⁴ are identical or different, substituted or unsubstituted, linked, unlinked or fused aryl or heteroaryl radicals and R⁹ = hydrogen or a substituted or unsubstituted alkyl or aryl radical which may contain one or more heteroatom(s).

2. Bisphosphite according to Claim 1, **characterized in that X** is a radical **Xa**, 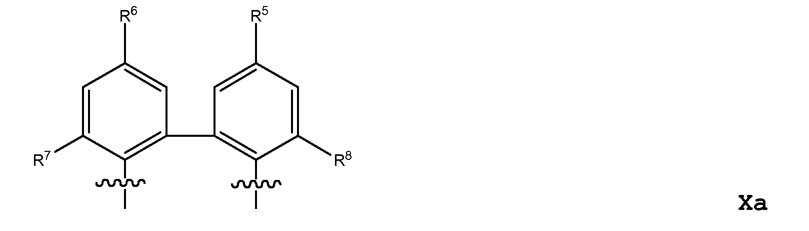 where the radicals R⁵, R⁶, R⁷ R⁸ can be, independently of one another, substituted or unsubstituted aliphatic, alicyclic, aromatic, heteroaromatic, mixed aliphatic-alicyclic, mixed aliphatic-aromatic, heterocyclic, mixed aliphatic-heterocyclic hydrocarbon radicals having from 1 to 50 carbon atoms or H, F, Cl, Br, I, -CF₃, -(CH₂)ᵢ(CF₂)ⱼCF₃ where i = 0-9 and j = 0-9, -SiR²¹₃, -Si(OR²¹)₃, -SiR²¹(OR²¹)₂, -SiR²¹₂OR²¹, -OSiR²¹₃, -OSi(OR²¹)₃, -OSiR²¹(OR²¹)₂, -OSiR²¹₂OR²¹, -OR¹⁹, -COR¹⁹, -CO₂R¹⁹, -CO₂M, -SO₂R¹⁹, -SOR¹⁹, -SO₃R¹⁹, -SO₃M, -SO₂NR¹⁹R²⁰, NR¹⁹R²⁰ or -N=CR¹⁹R²⁰, where R¹⁹, R²⁰ and R²¹ are selected independently from among H, monovalent substituted or unsubstituted aliphatic and aromatic hydrocarbon radicals having from 1 to 25 carbon atoms but R²¹ = H is excluded and M is an alkali metal ion, formally half an alkaline earth metal ion, an ammonium ion or a phosphonium ion.

3. Bisphosphite according to Claim 1, **characterized in that** X is an ethylene radical substituted by the radicals R^{1'}, R^{2'}, R^{3'} and R^{4'}.

4. Bisphosphite according to Claim 3, **characterized in that** X is an ethylene radical substituted by the radicals R^{1'}, R^{2'}, R^{3'} and R^{4'}, with the radicals R¹ and R^{1'}, R² and R^{2'}, R³ and R^{3'} and R⁴ and R^{4'} in each case being identical.

5. Bisphosphite according to any one of Claims 1 to 4, **characterized in that** the radicals R¹, R², R³, R⁴ are unsubstituted phenyl radicals.

6. Bisphosphite according to any one of Claims 1 to 5, **characterized in that** Y is selected from among bisphenoxy radicals of the formulae **IIa** to **IId** 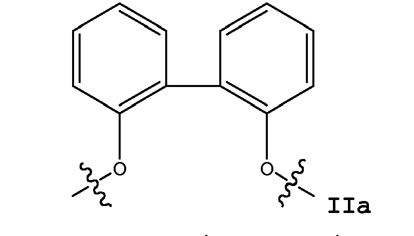 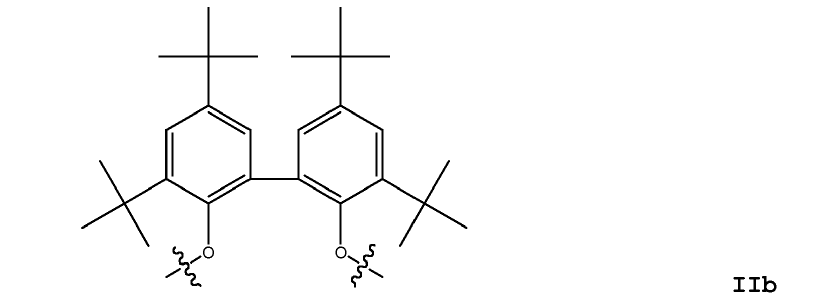 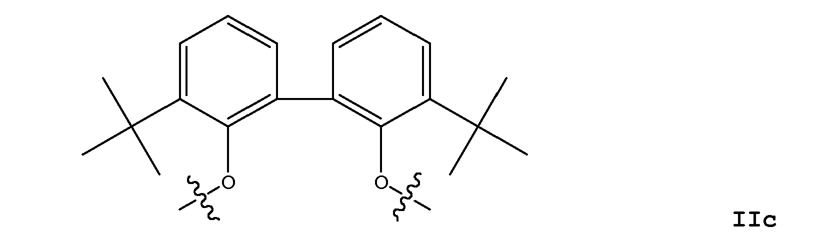 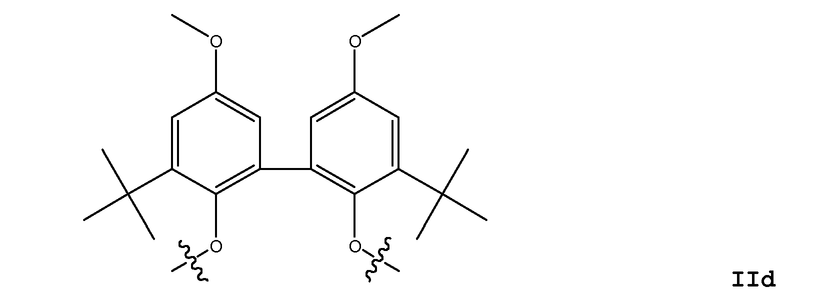 or bisnaphthoxy radicals of the formula **III**

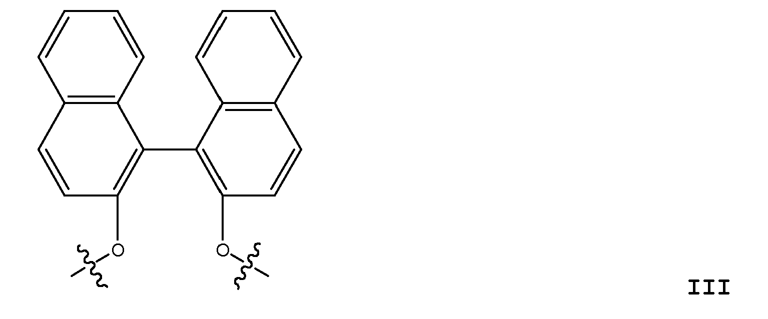

7. Phosphite-metal complex containing a metal of Group 4, 5, 6, 7, 8, 9 or 10 of the Periodic Table of the Elements and one or more bisphosphites according to any one of Claims 1 to 6.

8. Phosphite-metal complex according to Claim 7, **characterized in that** the metal is rhodium, palladium, nickel, platinum, cobalt or ruthenium.

9. Use of a bisphosphite of any of Claims 1 to 6 or a metal complex according to Claim 7 or 8 in catalysis.

10. Use according to Claim 9, **characterized in that** the catalysis is a homogeneous catalysis.

11. Use according to Claim 9 or 10, **characterized in that** the catalyzed reaction is the hydroformylation of olefins.

12. Use according to Claim 11, **characterized in that** a metal complex having rhodium or cobalt as metal is used in hydroformylation.

13. Use according to any one of Claims 11 and 12, **characterized in that** the molar ratio of metal to bisphosphite in the reaction mixture is from 1:1 to 1:500.

14. Process for preparing a bisphosphite according to any one of Claims 1 to 6, **characterized in that** it comprises the following steps
1) reaction of a diol **Aa** or an alcohol amine **Ab** with phosphorus trihalide to form an intermediate **A**,
2) reaction of a tetraarylethanediol **Ca**, which bears identical or different, substituted or unsubstituted, linked or unlinked, fused or unfused aryl or heteroaryl radicals, with phosphorus trihalide to form an intermediate **C**,
3) reaction of the intermediate **A** or **C** with a diol **Ba** to form an intermediate **B** and
4) reaction of the intermediate **B** with the intermediate **A** or **C** which was not used in process step 3).

15. Process according to Claim 14, **characterized in that** the diols **Aa**, **Ba**, **Ca** or the alcohol amine **Ab** and/or the intermediate **C** is/are reacted in steps 1), 2), 3) and/or 4) with a metal compound to form the corresponding metal salt which is then reacted with the phosphorus trihalide.

16. Process according to Claim 15, **characterized in that** sodium hydride, methyllithium or butyllithium is used as metal compound.

17. Process according to at least one of Claims 14 to 16, **characterized in that** the reaction with phosphorus trihalide is carried out in the presence of a base.

18. Process according to Claim 17, **characterized in that** a tertiary amine is used as base.

## Revendications

1. Bisphosphite de formule I 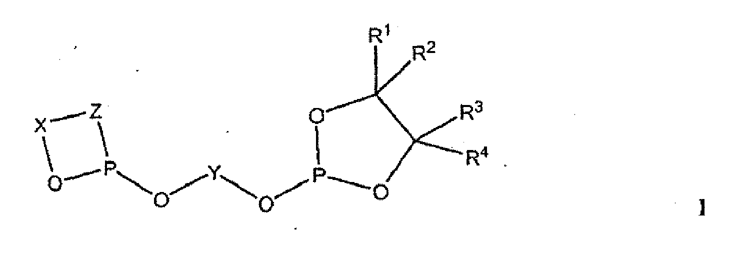 dans laquelle X est un radical bisalkylène ou bisaryle divalent, substitué ou non substitué, qui peut contenir un ou plusieurs hétéroatome(s), Y est un radical bisarylène ou bisalkyle divalent, substitué ou non substitué, qui peut contenir un ou plusieurs hétéroatome(s), Z est un atome d'oxygène ou NR⁹, R¹, R², R³, R⁴ sont des radicaux aryle ou hétéroaryle identiques ou différents, substitués ou non substitués, reliés, non reliés ou condensés, et R⁹ est un atome d'hydrogène ou un radical alkyle ou aryle substitué ou non substitué, qui peut contenir un ou plusieurs hétéroatome(s).

2. Bisphosphite selon la revendication 1, **caractérisé en ce que**
X est un radical Xa 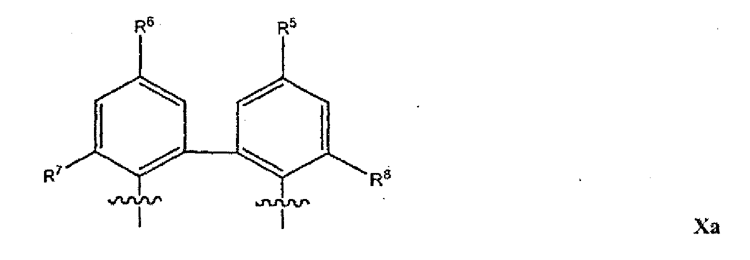 les radicaux R⁵, R⁶, R⁷, R⁸ représentant chacun indépendamment des autres des radicaux hydrocarbonés substitués ou non substitués, aliphatiques, alicycliques, aromatiques, hétéroaromatiques, aliphatiques-alicycliques en mélange, aliphatiques-aromatiques en mélange, hétérocycliques, aliphatiques-hérétocycliques en mélange, ayant 1 à 50 atomes de carbone, ou H, F, Cl, Br, I, -CF₃, -(CH₂)ᵢ(CF₂)ⱼCF₃, avec i = 0-9 et j = 0-9, -SiR²¹₃, -Si(OR²¹)₃, -SiR²¹(OR²¹)₂, -SiR²¹₂OR²¹, -OSiR²¹₃, -OSi(OR²¹)₃, -OSiR²¹(OR²¹)₂, -OSiR²¹₂OR²¹, -OR¹⁹, -COR¹⁹, -CO₂R¹⁹, -CO₂M, -SO₂R¹⁹, -SOR¹⁹, -SO₃R¹⁹, -SO₃M, -SO₂NR¹⁹R²⁰, -NR¹⁹R²⁰ ou -N-CR¹⁹R²⁰, R¹⁹, R²⁰ et R²¹ étant chacun indépendamment des autres choisis parmi H, les radicaux hydrocarbonés monovalents substitués ou n o n substitués, aliphatiques et aromatiques, ayant 1 à 25 atomes de carbone, mais cependant R²¹ ne peut être un atome d'hydrogène, et M est un ion d'un métal alcalin, et de par sa formule un semi-ion d'un métal alcalino-terreux, ammonium ou phosphonium.

3. Bisphosphite selon la revendication 1, **caractérisé en ce que** X est un radical éthylène substitué par les radicaux R^{1'}, R^{2'}, R^{3'} et R^{4'}.

4. Bisphosphite selon la revendication 3, **caractérisé en ce que** X est un radical éthylène substitué par les radicaux R^{1'}, R^{2'}, R^{3'} et R^{4'}, les radicaux R¹ et R^{1'}, R² et R^{2'}, R³ et R^{3'}, et R⁴ et R^{4'}, étant deux par deux égaux.

5. Bisphosphite selon l'une des revendications 1 à 4, **caractérisé en ce que** les radicaux R¹, R², R³, R⁴ sont des radicaux phényle non substitués.

6. Bisphosphite selon l'une des revendications 1 à 5, **caractérisé en ce que** Y est choisi parmi les radicaux bisphénoxy de formules IIa à IId 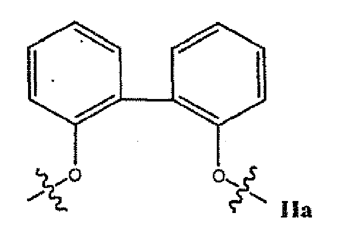 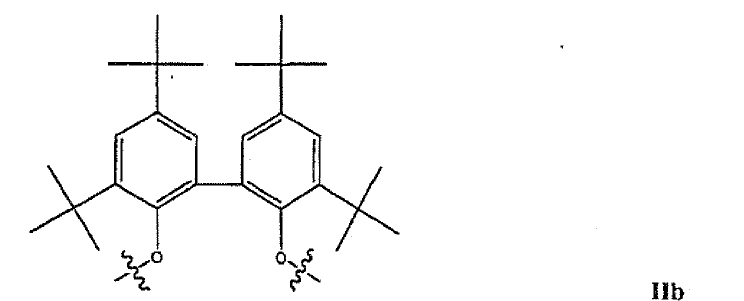 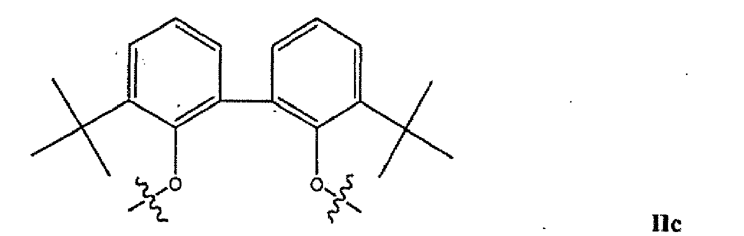 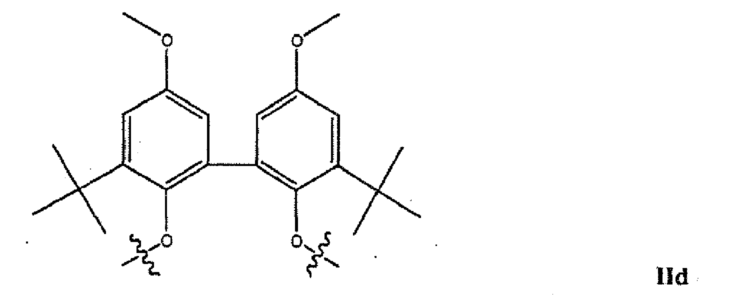 ou parmi les radicaux bisnaphtoxy de formule III

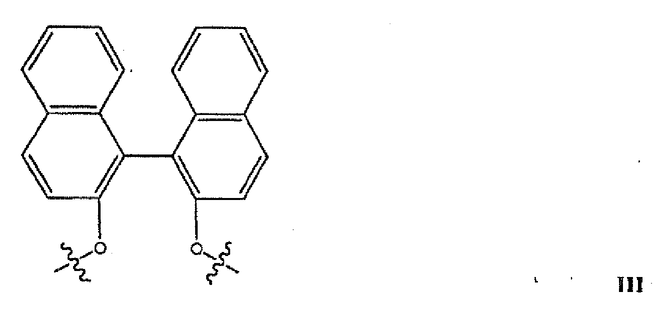

7. Complexe phosphite-métal, contenant un métal du 4ème, du 5ème, du 6ème, du 7ème, du 8ème, du 9ème ou du 10ème Groupe du Tableau Périodique des Eléments, et un ou plusieurs bisphosphites selon l'une des revendications 1 à 6.

8. Complexe phosphite-métal selon la revendication 7, **caractérisé en ce que** le métal est le rhodium, le palladium, le nickel, le platine, le cobalt ou le ruthénium.

9. Utilisation d'un bisphosphite des revendications 1 à 6 ou d'un complexe métallique selon la revendication 7 ou 8 en catalyse.

10. Utilisation selon la revendication 9, **caractérisée en ce que** la catalyse est une catalyse homogène.

11. Utilisation selon la revendication 9 ou 10, **caractérisée en ce que** la réaction catalysée est l'hydroformylation de l'oléfines.

12. Utilisation selon la revendication 11, **caractérisée en ce qu'**on utilise lors de l'hydroformylation un complexe métallique qui en tant que métal contient du rhodium ou du cobalt.

13. Utilisation selon l'une des revendications 11 ou 12, **caractérisée en ce que** le rapport en moles du métal au bisphosphite dans le mélange réactionnel est de 1:1 à 1:500.

14. Procédé de préparation d'un bisphosphite selon l'une des revendications 1 à 6, **caractérisé en ce qu'**il comprend les étapes suivantes :
1) réaction d'un diol Aa ou d'une alcoolamine Ab avec un trihalogénure de phosphore pour obtenir le produit intermédiaire A,
2) réaction d'un tétraaryléthanediol Ca, qui comprend des radicaux aryle ou hétéroaryle identiques ou différents, substitués ou non substitués, reliés ou non reliés, condensés ou non condensés, avec un trihalogénure de phosphore pour obtenir le produit intermédiaire C,
3) réaction du produit intermédiaire A ou C avec un diol Ba pour donner un produit intermédiaire B, et
4) réaction du produit intermédiaire B avec le produit intermédiaire A ou C qui n'a pas été utilisé dans l'étape 3).

15. Procédé selon la revendication 14, **caractérisé en ce que** les diols Aa, Ba, Ca, ou l'alcoolamine Ab, et/ou le produit intermédiaire C, sont dans les étapes 1), 2), 3) et/ou 4) mis à réagir avec un composé métallique pour donner le sel métallique correspondant, qui ensuite est mis à réagir avec le trihalogénure de phosphore.

16. Procédé selon la revendication 15, **caractérisé en ce qu'**on utilise en tant que composé métallique l'hydrure de sodium, le méthyllithium ou le butyllithium.

17. Procédé selon au moins l'une des revendications 14 à 16, **caractérisé en ce que** la réaction avec le trihalogénure de phosphore est mise en oeuvre en présence d'une base.

18. Procédé selon la revendication 17, **caractérisé en ce qu'**on utilise en tant que base une amine tertiaire.
